# EUROPEAN PATENT APPLICATION

(11) **EP 3 564 231 A1**
(43) Date of publication of application: **06.11.2019**
(21) Application number: 17885670.4
(22) Date of filing: 27.12.2017
(51) Int. Cl.: C07D 401/14, C07D 405/14, A61K 31/44, A61K 31/4196, A61P 15/00

(54) **AZABICYCLO-SUBSTITUTED TRIAZOLE DERIVATIVE, PREPARATION METHOD THEREOF, AND APPLICATION OF SAME IN MEDICINE**

(30) Priority: 28.12.2016 CN 201611233476
(71) Applicant: Jiangsu Hengrui Medicine Co., Ltd., Jiangsu 222047 (CN); Shanghai Hengrui Pharmaceutical Co., Ltd., Shanghai 200245 (CN)
(72) Inventor: LI, Xin, Shanghai 200245 (CN); CHEN, Yang, Shanghai 200245 (CN); LIU, Tao, Shanghai 200245 (CN); HE,Feng, Shanghai 200245 (CN); TAO, Weikang, Shanghai 200245 (CN)
(74) Representative: Potter Clarkson
(86) International application number: PCT/CN2017/118784
(87) International publication number: WO 2018/121551

(57) **Abstract**

The present invention relates to an azabicyclo-substituted triazole derivative, a preparation method thereof, and an application of the same in medicine. In particular, the present invention relates to a novel azabicyclo-substituted triazole derivative represented by general formula (I), a preparation method thereof, a pharmaceutical composition containing the derivative, a use thereof as a therapeutic agent, especially as an oxytocin antagonist, and a use of the same in preparing a drug for treating or preventing a disease or disorder known or shown to have beneficial effect thereon with oxytocin being suppressed. The definition of each substituent in the general formula (I) is the same as the definition in the specification.

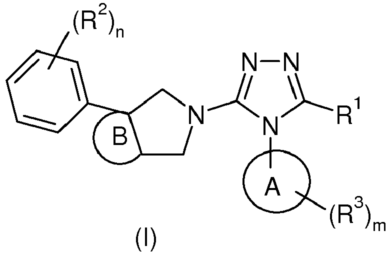

## Description

### FIELD OF THE INVENTION

The present invention belongs to the field of medicine, and relates to a novel azabicyclo-substituted triazole derivative, a method for preparing the same, a pharmaceutical composition comprising the same, a use thereof as a therapeutic agent, in particular as an oxytocin antagonist, and a use thereof in the preparation of a medicament for treating or preventing a disease or condition where inhibition of oxytocin is known, or can be shown, to produce a beneficial effect.

### BACKGROUND OF THE INVENTION

Oxytocin (OT) is a cyclic nonapeptide that is normally synthesized by the hypothalamic paraventricular nucleus and released via the posterior pituitary. OT has a wide range of physiological functions, including social connections, sexual reproduction, labor and the like. OT exerts physiological effects by binding to oxytocin receptors (OTRs).

In recent years, strong evidences have been accumulated, indicating that the hormone oxytocin plays a major role in initiating labor in mammals, in particular in humans. By "down-regulating" oxytocin, it is expected that both the direct (contractile) and indirect (increased prostaglandin synthesis) effects of oxytocin on the uterus could be blocked. An oxytocin modulator, e. g. blocker or antagonist would likely be efficacious for treating preterm labor. A further condition related to oxytocin is dysmenorrhea, which is characterized by pain and discomfort associated with menses. Oxytocin plays a role in dysmenorrhea due to its activity as a uterine vasoconstrictor (Akerlund et al., Ann. NY Acad. Sci. 734: 47-56, 1994). Oxytocin antagonists have a therapeutic efficacy on this condition.

It is well documented that the levels of circulating oxytocin increase during sexual stimulation and arousal, and peak during orgasm in both men and women. As detailed in Gimpl and Fahrenholz (Physiological Reviews 81(2): 629-683, 2001), oxytocin has been found to be one of the most potent agents to induce penile erection in rats, rabbits and monkeys. In addition, central administration of oxytocin is claimed to reduce the latency to achieve ejaculation and to shorten the post-ejaculatory interval. Likewise, Meston et al (Arch. Gen. Psychiatry, 57(11): 1012-30, 2000) states that in male animals, oxytocin facilitates penile erections when injected into specific areas of the brain (i.e., periventricular nucleus of the hypothalamus) and shortens the ejaculation latency and postejaculation interval when injected either centrally or peripherally. It has been well documented within the art that the administration of the oxytocin receptor agonist, i.e., 8-vasotocin, significantly reduces non-contact penile erections (see, for example, Melis et al., Neuro science Letters 265: 171-174, 1999).

The structure of oxytocin receptor is very similar to that of vasopressin receptors (including V1a receptor, V1b receptor, V2 receptor). V1a receptor and V2 receptor are mainly expressed in the periphery, which regulate blood pressure and kidney function, respectively. V1b receptor is mainly expressed in the brain and pituitary gland, and can control the release of adrenocorticotropic hormone and β-endorphin. Therefore, for safety reasons, highly selective OTR agonists are key issues that must be considered in future development (Alan D. Borthwick. J. Med. Chem. 2010, 53, 6525-6538).

A series of patent applications of OTR antagonists are currently disclosed, including WO2005028452, WO2005082866, WO2006077496, WO2006092731, WO2006100588 and WO2006100557. However, a highly selective OTR antagonist is still the focus of development. The inventor designs a compound having a structure of formula (I) by continuous efforts, and finds that a compound having such a structure has a highly selective inhibition effect on OTR.

### SUMMARY OF THE INVENTION

The object of the present invention is to provide a compound of formula (I), or a tautomer, mesomer, racemate, enantiomer, diastereomer thereof, or mixture thereof, or a pharmaceutically acceptable salt thereof, 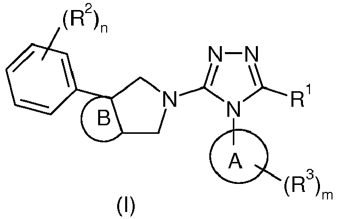 wherein:
ring A is aryl or heteroaryl;
ring B is cycloalkyl or heterocyclyl;
R¹ is alkyl or cycloalkyl, wherein the alkyl is optionally substituted by one or more substituents selected from the group consisting of alkoxy, halogen, haloalkyl, haloalkoxy, deuterated alkoxy, hydroxy, hydroxyalkyl, cyano, amino, nitro, cycloalkyl, heterocyclyl, heterocyclyloxy, aryl, heteroaryl and -OR⁴;
each R² is identical or different and each is independently selected from the group consisting of hydrogen, halogen, alkyl, alkoxy, haloalkyl, hydroxy, hydroxyalkyl, cyano, amino, nitro, cycloalkyl and heterocyclyl;
each R³ is identical or different and each is independently selected from the group consisting of hydrogen, halogen, alkyl, alkoxy, haloalkyl, hydroxy, hydroxyalkyl, cyano, amino, nitro, cycloalkyl and heterocyclyl;
R⁴ is selected from the group consisting of hydroxyalkyl, cycloalkyl, aryl and heteroaryl;
n is 0, 1, 2, 3, 4 or 5; and
m is 0, 1, 2, 3 or 4.

In a preferred embodiment of the present invention, the compound of formula (I) is a compound of formula (II): 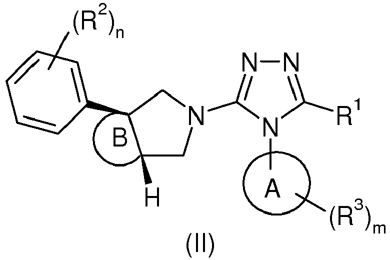 or a tautomer, mesomer, racemate, enantiomer, diastereomer thereof, or mixture thereof, or a pharmaceutically acceptable salt thereof,
ring A, ring B, R¹-R³, n and m are as defined in formula (I).

In a preferred embodiment of the present invention, in the compound of formula (I), ring B is 3-5 membered cycloalkyl or heterocyclyl, and preferably cyclopropyl.

In a preferred embodiment of the present invention, the compound of formula (I) or (II) is a compound of formula (III): 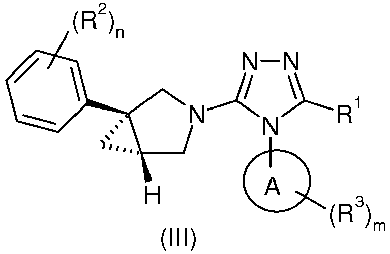 or a tautomer, mesomer, racemate, enantiomer, diastereomer thereof, or mixture thereof, or a pharmaceutically acceptable salt thereof,
wherein:
ring A, R¹-R³, n and m are as defined in formula (I).

In a preferred embodiment of the present invention, in the compound of formula (I), ring A is pyridyl or benzodioxol, and preferably

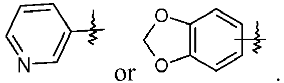

In a preferred embodiment of the present invention, in the compound of formula (I), R¹ is alkyl or cycloalkyl, wherein the alkyl is optionally substituted by one or more substituents selected from the group consisting of halogen, cyano, alkoxy, haloalkoxy, deuterated alkoxy and heterocyclyloxy.

In a preferred embodiment of the present invention, in the compound of formula (I), each R² is identical or different and each is independently selected from the group consisting of hydrogen, halogen and alkyl.

In a preferred embodiment of the present invention, in the compound of formula (I), R³ is alkoxy.

In a preferred embodiment of the present invention, in the compound of formula (I), n is 2; and m is 0 or 1.

The compound of the present invention includes all conformational isomers thereof, e.g., cis-isomers and trans-isomers; and all optical isomers and stereoisomers as well as mixtures thereof. The compound of the present invention has asymmetric centers, and therefore there are different enantiomeric and diastereomeric isomers. The present invention relates to a use of the compound of the present invention, and all pharmaceutical compositions applying and comprising the same, and a therapeutic method thereof. The present invention relates to a use of all such isomers and mixtures thereof.

Typical compounds of the present invention include, but are not limited to:

| Example No. | Structure and name of the compound |
|---|---|
| 1 | 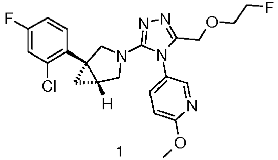 |
| | (1*S*,5*R*)-1-(2-Chloro-4-fluorophenyl)-3-(5-((2-fluoroethoxy)methyl)-4-(6-me thoxypyridin-3-yl)-4*H*-1,2,4-triazol-3-yl)-3-azabicyclo[3.1.0]hexane **1** |
| 2 | 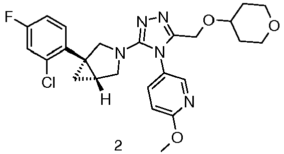 |
| | (1*S*,5*R*)-1-(2-Chloro-4-fluorophenyl)-3-(4-(6-methoxypyridin-3-yl)-5-(((tetra hydro-2*H*-pyran-4-yl)oxy)methyl)-4H-1,2,4-triazol-3-yl)-3-azabicyclo[3.1.0] hexane **2** |
| 3 | 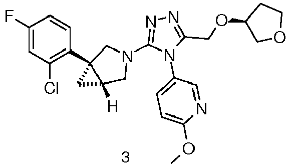 |
| | (1*S*,5*R*)-1-(2-Chloro-4-fluorophenyl)-3-(4-(6-methoxypyridin-3-yl)-5-((((*S*)-t etrahydrofuran-3-yl)oxy)methyl)-4*H*-1,2,4-triazol-3-yl)-3-azabicyclo[3.1.0]h exane **3** |
| 4 | 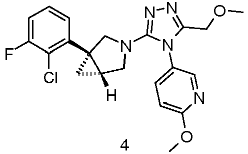 |
| | (1*S*,5*R*)-1-(2-Chloro-3-fluorophenyl)-3-(5-(methoxymethyl)-4-(6-metho xypyridin-3-yl)-4*H*-1,2,4-triazol-3-yl)-3-azabicyclo[3.1.0]hexane **4** |
| 5 | 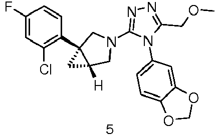 |
| | (1*S*,5*R*)-3-(4-(Benzo[*d*][1,3]dioxol-5-yl)-5-(methoxymethyl)-4*H*-1,2,4-t riazol-3-yl)-1-(2-chloro-4-fluorophenyl)-3-azabicyclo[3.1.0]hexane **5** |
| 6 | 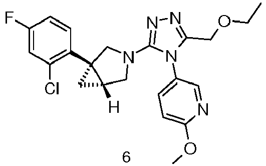 |
| | (1*S*,5*R*)-1-(2-Chloro-4-fluorophenyl)-3-(5-(ethoxymethyl)-4-(6-methoxypyri din-3-yl)-4*H*-1,2,4-triazol-3-yl)-3-azabicyclo[3.1.0]hexane **6** |
| 7 | 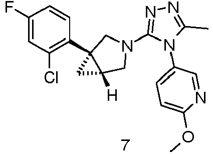 |
| | (1*S*,5*R*)-1-(2-Chloro-4-fluorophenyl)-3-(4-(6-methoxypyridin-3-yl)-5-methyl -4*H*-1,2,4-triazol-3-yl)-3-azabicyclo[3.1.0]hexane **7** |
| 8 | 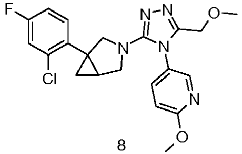 |
| | 1-(2-Chloro-4-fluorophenyl)-3-(5-(methoxymethyl)-4-(6-methoxypyridin-3-yl)-4*H*-1,2,4-triazol-3-yl)-3-azabicyclo[3.1.0]hexane **8** |
| 9 | 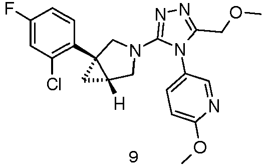 |
| | (1*S*,5*R*)-1-(2-Chloro-4-fluorophenyl)-3-(5-(methoxymethyl)-4-(6-methoxypy ridin-3-yl)-4*H*-1,2,4-triazol-3-yl)-3-azabicyclo[3.1.0]hexane **9** |
| 10 | 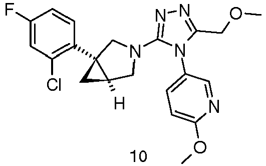 |
| | (1*R*,5*S*)-1-(2-Chloro-4-fluorophenyl)-3-(5-(methoxymethyl)-4-(6-methoxypy ridin-3-yl)-4*H*-1,2,4-triazol-3-yl)-3-azabicyclo[3.1.0]hexane **10** |
| 11 | 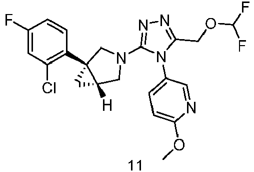 |
| | (1*S*,5*R*)-1-(2-Chloro-4-fluorophenyl)-3-(5-((difluoromethoxy)methyl)-4-(6-methoxypyridin-3-yl)-4*H*-1,2,4-triazol-3-yl)-3-azabicyclo[3.1.0]hexane **11** |
| 12 | 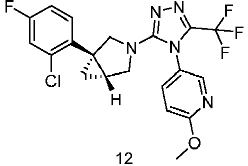 |
| | (1*S*,5*R*)-1-(2-Chloro-4-fluorophenyl)-3-(4-(6-methoxypyridin-3-yl)-5-(trifluo romethyl)-4*H*-1,2,4-triazol-3-yl)-3-azabicyclo[3.1.0]hexane **12** |
| 13 | 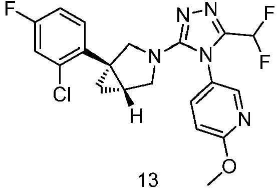 |
| | (1*S*,5*R*)-1-(2-Chloro-4-fluorophenyl)-3-(5-(difluoromethyl)-4-(6-methoxypyr idin-3-yl)-4*H*-1,2,4-triazol-3-yl)-3-azabicyclo[3.1.0]hexane **13** |
| 14 | 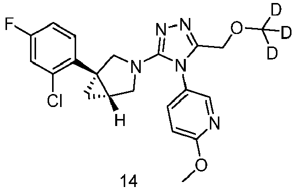 |
| | (1*S*,5*R*)-1-(2-Chloro-4-fluorophenyl)-3-(5-((methoxy-*d*₃)methyl)-4-(6-metho xypyridin-3-yl)-4*H*-1,2,4-triazol-3-yl)-3-azabicyclo[3.1.0]hexane **14** |
| 15 | 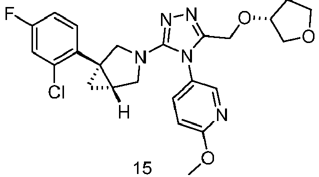 |
| | (1*S*,5*R*)-1-(2-Chloro-4-fluorophenyl)-3-(4-(6-methoxypyridin-3-yl)-5-((((*R*)-tetrahydrofuran-3-yl)oxy)methyl)-4*H*-1,2,4-triazol-3-yl)-3-azabicyclo[3.1.0] hexane **15** |
| 16 | 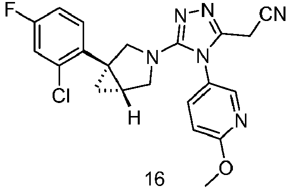 |
| | (1*S*,5*R*)-1-(2-Chloro-4-fluorophenyl)-3-(4-(6-methoxypyridin-3-yl)-5-cyano methyl-4*H*-1,2,4-triazol-3-yl)-3-azabicyclo[3.1.0]hexane **16** |
| 17 | 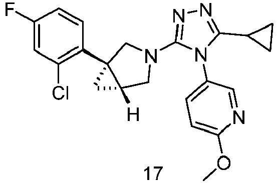 |
| | (1*S*,5*R*)-1-(2-Chloro-4-fluorophenyl)-3-(5-cyclopropyl-4-(6-methoxypyridin-3-yl)-4*H*-1,2,4-triazol-3-yl)-3-azabicyclo[3.1.0]hexane **17** |

or a tautomer, mesomer, racemate, enantiomer, diastereomer thereof, or mixture thereof, or a pharmaceutically acceptable salt thereof.

A preferred embodiment of the present invention relates to a compound of formula (I-A) which is an intermediate for preparing the compound of formula (I): 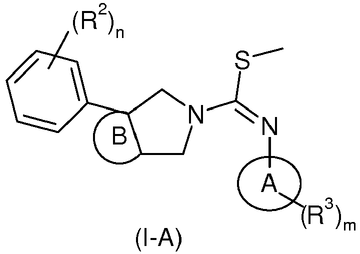 or a tautomer, mesomer, racemate, enantiomer, diastereomer thereof, or mixture thereof, or a pharmaceutically acceptable salt thereof,
wherein:
ring A, ring B, R², R³, n and m are as defined in formula (I).

The compounds of formula (I-A) include, but are not limited to:

| Example No. | Structure and name of the compound |
|---|---|
| 1h | 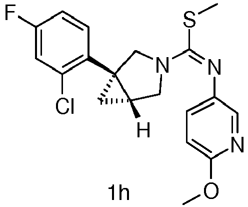 |
| | Methyl (1*S*,5*R*,*E*)-1-(2-chloro-4-fluorophenyl)-*N*-(6-methoxypyridin-3-yl)-3-azabicyclo[3.1.0]hexane-3-carbimidothioate **1h** |
| 4h | 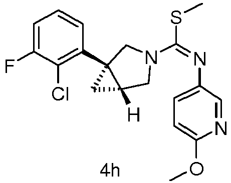 |
| | Methyl (1*S*,5*R*,*E*)-1-(2-chloro-3-fluorophenyl)-*N*-(6-methoxypyridin-3-yl)-3-azabicyclo[3.1.0]hexane-3-carbimidothioate **4h** |
| 5c | 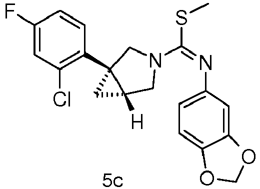 |
| | Methyl (1*S*,5*R*,*E*)-*N*-benzo[*d*][1,3]dioxol-5-yl-1-(2-chloro-4-fluorophenyl)-3-azabicyclo[3.1.0]hexane-3-carbimidothioate **5c** |
| 8i | 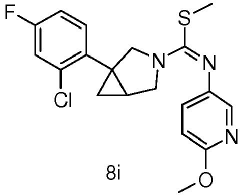 |
| | Methyl (*E*)-1-(2-chloro-4-fluorophenyl)-*N*-(6-methoxypyridin-3-yl)-3-azabicyclo[3.1.0]hexane-3-carbimidothioate **8i** |
| 10f | 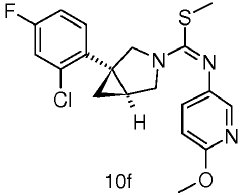 |
| | Methyl (1*R*,5*S*,*E*)-1-(2-chloro-4-fluorophenyl)-*N*-(6-methoxypyridin-3-yl)-3-azabicyclo[3.1.0]hexane-3-carbimidothioate **10f** |

In another aspect, the present invention relates to a method for preparing the compound of formula (I), comprising a step of: 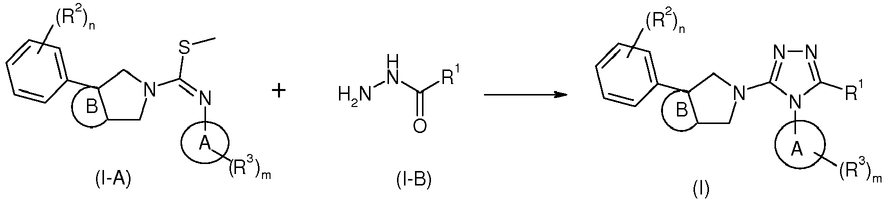 heating a compound of formula (I-A) and a compound of formula (I-B) or a hydrochloride salt thereof under an acidic condition to obtain the compound of formula (I),
wherein:
ring A, ring B, R¹-R³, n and m are as defined in formula (I).

In another aspect, the present invention relates to a method for preparing the compound of formula (III), comprising a step of: 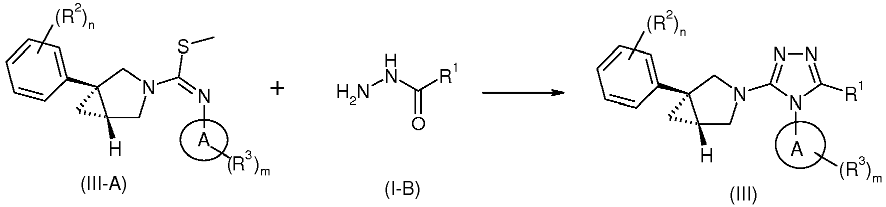 heating a compound of formula (III-A) and a compound of formula (I-B) or a hydrochloride salt thereof under an acidic condition to obtain the compound of formula (III),
wherein:
ring A, R¹-R³, n and m are as defined in formula (I).

In another aspect, the present invention relates to a pharmaceutical composition comprising a therapeutically effective amount of the compound of formula (I), or a tautomer, mesomer, racemate, enantiomer, diastereomer thereof, or mixture thereof, or a pharmaceutically acceptable salt thereof, and one or more pharmaceutically acceptable carriers, diluents or excipients. The present invention also relates to a method for preparing the aforementioned composition, comprising a step of mixing the compound of formula (I), or a tautomer, mesomer, racemate, enantiomer, diastereomer thereof, or mixture thereof, or a pharmaceutically acceptable salt thereof, with one or more pharmaceutically acceptable carriers, diluents or excipients.

The present invention further relates to a use of the compound of formula (I), or a tautomer, mesomer, racemate, enantiomer, diastereomer thereof, or mixture thereof, or a pharmaceutically acceptable salt thereof, or the pharmaceutical composition comprising the same, in the preparation of a medicament for treating or preventing a disease or condition where inhibition of oxytocin is known, or can be shown, to produce a beneficial effect, wherein the disease or condition is selected from the group consisting of sexual dysfunction, male sexual dysfunction, female sexual dysfunction, hypoactive sexual desire disorder, sexual arousal disorder, orgasmic disorder, sexual pain disorder, premature ejaculation, preterm labour, complications in labour, appetite and feeding disorders, benign prostatic hyperplasia, premature birth, dysmenorrhea, congestive heart failure, arterial hypertension, liver cirrhosis, nephrotic hypertension, ocular hypectension, obsessive compulsive disorder and neuropsychiatric disorders, and more preferably selected from the group consisting of sexual arousal disorder, orgasmic disorder, sexual pain disorder and premature ejaculation.

The present invention further relates to a use of the compound of formula (I), or a tautomer, mesomer, racemate, enantiomer, diastereomer thereof, or mixture thereof, or a pharmaceutically acceptable salt thereof, or the pharmaceutical composition comprising the same, in the preparation of a medicament for antagonizing oxytocin.

The present invention further relates to a method for treating or preventing a disease or condition where inhibition of oxytocin is known, or can be shown, to produce a beneficial effect, comprising a step of administering to a patient in need thereof a therapeutically effective amount of the compound of formula (I), or a tautomer, mesomer, racemate, enantiomer, diastereomer thereof, or mixture thereof, or a pharmaceutically acceptable salt thereof, or the pharmaceutical composition comprising the same.

The present invention further relates to a method for treating or preventing a disease selected from the group consisting of sexual dysfunction, male sexual dysfunction, female sexual dysfunction, hypoactive sexual desire disorder, sexual arousal disorder, orgasmic disorder, sexual pain disorder, premature ejaculation, preterm labour, complications in labour, appetite and feeding disorders, benign prostatic hyperplasia, premature birth, dysmenorrhea, congestive heart failure, arterial hypertension, liver cirrhosis, nephrotic hypertension, ocular hypectension, obsessive compulsive disorder and neuropsychiatric disorders, and preferably selected from the group consisting of sexual dysfunction, sexual arousal disorder, orgasmic disorder, sexual pain disorder and premature ejaculation, comprising a step of administering to a patient in need thereof a therapeutically effective amount of the compound of formula (I), or a tautomer, mesomer, racemate, enantiomer, diastereomer thereof, or mixture thereof, or a pharmaceutically acceptable salt thereof, or the pharmaceutical composition comprising the same.

The present invention further relates to a method for antagonizing oxytocin, comprising a step of administering to a patient in need thereof a therapeutically effective amount of the compound of formula (I), or a tautomer, mesomer, racemate, enantiomer, diastereomer thereof, or mixture thereof, or a pharmaceutically acceptable salt thereof, or the pharmaceutical composition comprising the same.

The present invention further relates to the compound of formula (I), or a tautomer, mesomer, racemate, enantiomer, diastereomer thereof, or mixture thereof, or a pharmaceutically acceptable salt thereof, or the pharmaceutical composition comprising the same, for use as a medicament.

The present invention further relates to the compound of formula (I), or a tautomer, mesomer, racemate, enantiomer, diastereomer thereof, or mixture thereof, or a pharmaceutically acceptable salt thereof, or the pharmaceutical composition comprising the same, for use as an oxytocin antagonist.

The present invention further relates to the compound of formula (I), or a tautomer, mesomer, racemate, enantiomer, diastereomer thereof, or mixture thereof, or a pharmaceutically acceptable salt thereof, or the pharmaceutical composition comprising the same, for use in treating or preventing a disease selected from the group consisting of sexual dysfunction, male sexual dysfunction, female sexual dysfunction, hypoactive sexual desire disorder, sexual arousal disorder, orgasmic disorder, sexual pain disorder, premature ejaculation, preterm labour, complications in labour, appetite and feeding disorders, benign prostatic hyperplasia, premature birth, dysmenorrhea, congestive heart failure, arterial hypertension, liver cirrhosis, nephrotic hypertension, ocular hypectension, obsessive compulsive disorder and neuropsychiatric disorders, and preferably selected from the group consisting of sexual arousal disorder, orgasmic disorder, sexual pain disorder and premature ejaculation.

Pharmaceutical compositions containing the active ingredient can be in a form suitable for oral administration, for example, a tablet, troche, lozenge, aqueous or oily suspension, dispersible powder or granule, emulsion, hard or soft capsule, or syrup or elixir. Oral compositions can be prepared according to any known method in the art for the preparation of pharmaceutical composition. Such composition can contain one or more ingredients selected from the group consisting of sweeteners, flavoring agents, colorants and preservatives, in order to provide a pleasing and palatable pharmaceutical preparation. Tablets contain the active ingredient in admixture with nontoxic pharmaceutically acceptable excipients suitable for the manufacture of tablets.

An aqueous suspension contains the active ingredient in admixture with excipients suitable for the manufacture of an aqueous suspension. The aqueous suspension can also contain one or more preservatives, one or more colorants, one or more flavoring agents, and one or more sweeteners.

An oil suspension can be formulated by suspending the active ingredient in a vegetable oil or mineral oil. The oil suspension can contain a thickener. The aforementioned sweeteners and flavoring agents can be added to provide a palatable formulation. These compositions can be preserved by adding an antioxidant.

The active ingredient in admixture with the dispersants or wetting agents, suspending agent or one or more preservatives can be prepared as a dispersible powder or granule suitable for the preparation of an aqueous suspension by adding water. Suitable dispersants or wetting agents and suspending agents are exemplified by those already mentioned above. Additional excipients, such as sweeteners, flavoring agents and colorants, can also be added. These compositions can be preserved by adding an antioxidant, such as ascorbic acid.

The pharmaceutical composition of the present invention can also be in the form of an oil-in-water emulsion. The oil phase can be a vegetable oil or mineral oil or mixture thereof. Suitable emulsifying agents can be naturally occurring phosphatides. The emulsion can also contain sweeteners, flavoring agents, preservatives and antioxidants. Such formulations can also contain demulcents, preservatives, colorants, and antioxidants.

The pharmaceutical composition of the present invention can be in the form of a sterile injectable aqueous solution. Acceptable vehicles or solvents that can be used are water, Ringer's solution or isotonic sodium chloride solution. The sterile injectable formulation can be a sterile injectable oil-in-water micro-emulsion in which the active ingredient is dissolved in the oil phase. The injectable solution or micro-emulsion can be introduced into a patient's bloodstream by local bolus injection. Alternatively, the solution and micro-emulsion are preferably administered in a manner that maintains a constant circulating concentration of the compound of the present invention. In order to maintain this constant concentration, a continuous intravenous delivery device can be used. An example of such a device is Deltec CADD-PLUS. TM. 5400 intravenous injection pump.

The pharmaceutical composition of the present invention can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic parenterally acceptable diluent or solvent. Moreover, sterile fixed oils can easily be used as a solvent or suspending medium. For this purpose, any blending fixed oils including synthetic mono- or di-glyceride can be employed. Moreover, fatty acids can also be employed in the preparation of an injection.

The compound of the present invention can be administered in the form of a suppository for rectal administration. These pharmaceutical compositions can be prepared by mixing the drug with a suitable non-irritating excipient that is solid at ordinary temperatures, but liquid in the rectum, thereby melting in the rectum to release the drug.

It is well known to those skilled in the art that the dosage of a drug depends on a variety of factors including but not limited to, the following factors: activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound of formula (I) or the type of pharmaceutically acceptable salt thereof can be verified by traditional therapeutic regimens.

### DETAILED DESCRIPTION OF THE INVENTION

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, an alkyl group is a lower alkyl having 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl group can be substituted or unsubstituted. When substituted, the substituent group(s) can be substituted at any available connection point. The substituent group(s) is preferably one or more groups independently optionally selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heteroalkoxy, cycloalkylthio, heterocyclylthio and -OR⁴.

The term "cycloalkyl" refers to a saturated or partially unsaturated monocyclic or polycyclic hydrocarbon substituent group having 3 to 20 carbon atoms, preferably 3 to 12 carbon atoms, more preferably 3 to 10 carbon atoms, and most preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like, and preferably cyclopropyl, cyclobutyl, cyclopentyl and cyclohexyl. Polycyclic cycloalkyl includes a cycloalkyl having a spiro ring, fused ring or bridged ring.

The term "spiro cycloalkyl" refers to a 5 to 20 membered polycyclic group with monocyclic rings connected through one shared carbon atom (called a spiro atom), wherein the rings can contain one or more double bonds, but none of the rings has a completely conjugated π-electron system. The spiro cycloalkyl is preferably 6 to 14 membered spiro cycloalkyl, and more preferably 7 to 10 membered spiro cycloalkyl. According to the number of the spiro atoms shared between the rings, the spiro cycloalkyl can be divided into mono-spiro cycloalkyl, di-spiro cycloalkyl, or poly-spiro cycloalkyl, and the spiro cycloalkyl is preferably a mono-spiro cycloalkyl or di-spiro cycloalkyl, and more preferably 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

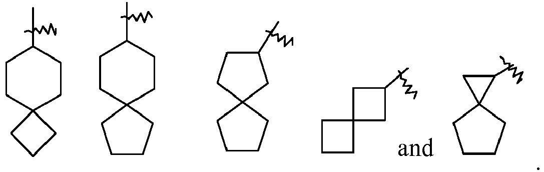

The term "fused cycloalkyl" refers to a 5 to 20 membered all-carbon polycyclic group, wherein each ring in the system shares an adjacent pair of carbon atoms with another ring, wherein one or more rings can contain one or more double bonds, but none of the rings has a completely conjugated π-electron system. The fused cycloalkyl is preferably 6 to 14 membered fused cycloalkyl, and more preferably 7 to 10 membered fused cycloalkyl. According to the number of membered rings, the fused cycloalkyl can be divided into bicyclic, tricyclic, tetracyclic or polycyclic fused cycloalkyl, and the fused cycloalkyl is preferably bicyclic or tricyclic fused cycloalkyl, and more preferably 5-membered/5-membered, or 5-membered/6-membered bicyclic fused cycloalkyl. Non-limiting examples of fused cycloalkyl include:

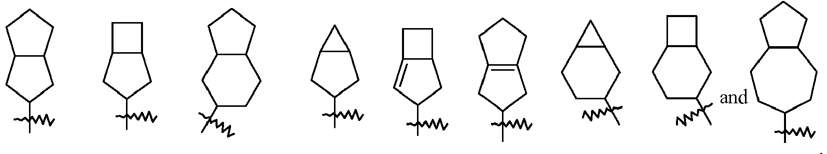

The term "bridged cycloalkyl" refers to a 5 to 20 membered all-carbon polycyclic group, wherein every two rings in the system share two disconnected carbon atoms, wherein the rings can have one or more double bonds, but none of the rings has a completely conjugated π-electron system. The bridged cycloalkyl is preferably 6 to 14 membered bridged cycloalkyl, and more preferably 7 to 10 membered bridged cycloalkyl. According to the number of membered rings, the bridged cycloalkyl can be divided into bicyclic, tricyclic, tetracyclic or polycyclic bridged cycloalkyl, and the bridged cycloalkyl is preferably bicyclic, tricyclic or tetracyclic bridged cycloalkyl, and more preferably bicyclic or tricyclic bridged cycloalkyl. Non-limiting examples of bridged cycloalkyls include:

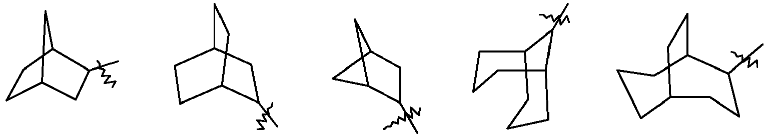

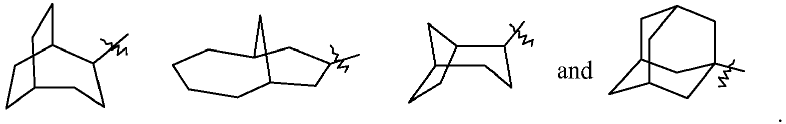

The ring of cycloalkyl can be fused to the ring of aryl, heteroaryl or heterocyclyl, wherein the ring bound to the parent structure is cycloalkyl. Non-limiting examples include indanyl, tetrahydronaphthyl, benzocycloheptyl and the like. The cycloalkyl can be optionally substituted or unsubstituted. When substituted, the substituent group(s) is preferably one or more group(s) independently optionally selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heteroalkoxy, cycloalkylthio, heterocyclylthio and -OR⁴.

The term "heterocyclyl" refers to a 3 to 20 membered saturated or partially unsaturated monocyclic or polycyclic hydrocarbon group, wherein one or more ring atoms are heteroatoms selected from the group consisting of N, O and S(O)ₜ (wherein t is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably, the heterocyclyl has 3 to 10 ring atoms, and most preferably 3 to 6 ring atoms. Non-limiting examples of monocyclic heterocyclyl include oxetanyl, azetidinyl, tetrahydrofuranyl, tetrahydropyranyl, pyrrolyl, piperidinyl, piperazinyl, morpholinyl, thiomorpholinyl, homopiperazinyl and the like, and preferably azetidinyl, oxetanyl, pyrrolyl and piperidinyl. Polycyclic heterocyclyl includes a heterocyclyl having a spiro ring, fused ring or bridged ring.

The term "spiro heterocyclyl" refers to a 5 to 20 membered polycyclic heterocyclyl group with monocyclic rings connected through one shared atom (called a spiro atom), wherein one or more ring atoms are heteroatoms selected from the group consisting of N, O and S(O)ₜ (wherein t is an integer of 0 to 2), with the remaining ring atoms being carbon atoms, where the rings can contain one or more double bonds, but none of the rings has a completely conjugated π-electron system. The spiro heterocyclyl is preferably 6 to 14 membered spiro heterocyclyl, and more preferably 7 to 10 membered spiro heterocyclyl. According to the number of the spiro atoms shared between the rings, the spiro heterocyclyl can be divided into mono-spiro heterocyclyl, di-spiro heterocyclyl, or poly-spiro heterocyclyl, and the spiro heterocyclyl is preferably mono-spiro heterocyclyl or di-spiro heterocyclyl, and more preferably 4-membered/4-membered, 4-membered/5-membered, 4-membered/6-membered, 5-membered/5-membered, or 5-membered/6-membered mono-spiro heterocyclyl. Non-limiting examples of spiro heterocyclyls include:

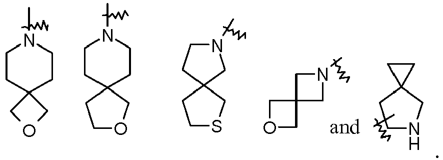

The term "fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated π-electron system, and wherein one or more ring atoms are heteroatoms selected from the group consisting of N, O and S(O)ₜ (wherein t is an integer of 0 to 2), with the remaining ring atoms being carbon atoms. The fused heterocyclyl is preferably 6 to 14 membered fused heterocyclyl, and more preferably 7 to 10 membered fused heterocyclyl. According to the number of membered rings, the fused heterocyclyl can be divided into bicyclic, tricyclic, tetracyclic or polycyclic fused heterocyclyl, and the fused heterocyclyl is preferably bicyclic or tricyclic fused heterocyclyl, and more preferably 5-membered/3-membered, 5-membered/4-membered or 5-membered/5-membered bicyclic fused heterocyclyl. Non-limiting examples of fused heterocyclyl include:

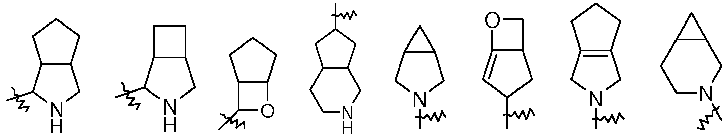

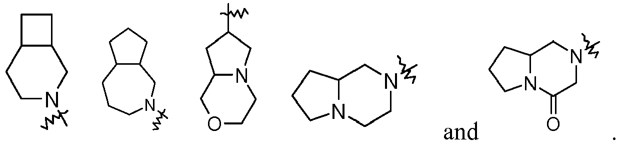

The term "bridged heterocyclyl" refers to a 5 to 14 membered polycyclic heterocyclyl group, wherein every two rings in the system share two disconnected atoms, wherein the rings can have one or more double bonds, but none of the rings has a completely conjugated π-electron system, and wherein one or more ring atoms are heteroatoms selected from the group consisting of N, O and S(O)ₜ (wherein t is an integer of 0 to 2), with the remaining ring atoms being carbon atoms. The bridged heterocyclyl is preferably 6 to 14 membered bridged heterocyclyl, and more preferably 7 to 10 membered bridged heterocyclyl. According to the number of membered rings, the bridged heterocyclyl can be divided into bicyclic, tricyclic, tetracyclic or polycyclic bridged heterocyclyl, and the bridged heterocyclyl is preferably bicyclic, tricyclic or tetracyclic bridged heterocyclyl, and more preferably bicyclic or tricyclic bridged heterocyclyl. Non-limiting examples of bridged heterocyclyls include:

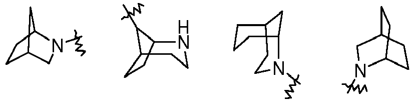

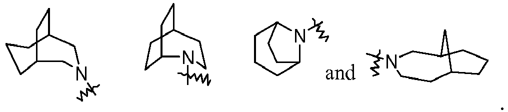

The ring of heterocyclyl can be fused to the ring of aryl, heteroaryl or cycloalkyl, wherein the ring bound to the parent structure is heterocyclyl. Non-limiting examples thereof include:

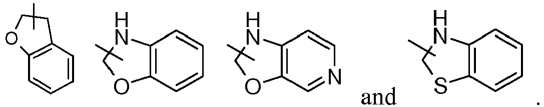

The heterocyclyl can be optionally substituted or unsubstituted. When substituted, the substituent group(s) is preferably one or more group(s) independently optionally selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heteroalkoxy, cycloalkylthio, heterocyclylthio and -OR⁴.

The term "aryl" refers to a 6 to 14 membered all-carbon monocyclic ring or polycyclic fused ring (i.e. each ring in the system shares an adjacent pair of carbon atoms with another ring in the system) having a conjugated π-electron system, preferably 6 to 10 membered aryl, for example, phenyl and naphthyl. The ring of aryl can be fused to the ring of heteroaryl, heterocyclyl or cycloalkyl, wherein the ring bound to the parent structure is aryl ring. Non-limiting examples thereof include:

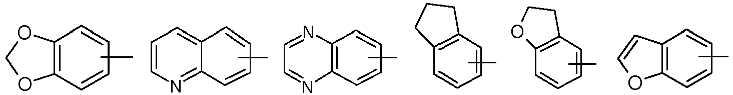

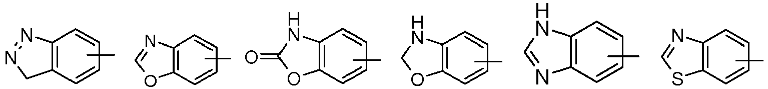

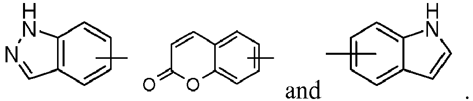

The aryl can be substituted or unsubstituted. When substituted, the substituent group(s) is preferably one or more group(s) independently optionally selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heteroalkoxy, cycloalkylthio, heterocyclylthio and -OR⁴.

The term "heteroaryl" refers to a 5 to 14 membered heteroaromatic system having 1 to 4 heteroatoms selected from the group consisting of O, S and N. The heteroaryl is preferably 5 to 10 membered heteroaryl, more preferably 5 or 6 membered heteroaryl, for example, furanyl, thienyl, pyridyl, pyrrolyl, N-alkylpyrrolyl, pyrimidinyl, pyrazinyl, imidazolyl, pyrazolyl, tetrazolyl and the like, and preferably pyridyl. The ring of heteroaryl can be fused to the ring of aryl, heterocyclyl or cycloalkyl, wherein the ring bound to the parent structure is heteroaryl ring. Non-limiting examples thereof include:

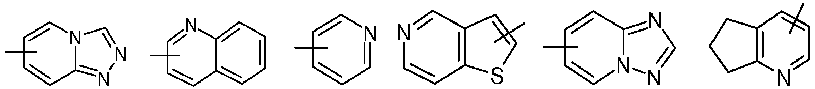

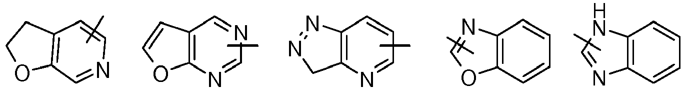

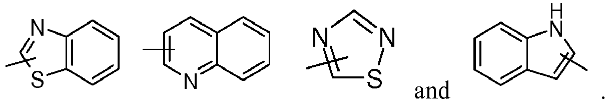

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heteroalkoxy, cycloalkylthio, heterocyclylthio and -OR⁴.

The term "alkoxy" refers to an -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, amino, nitro, cyano, cycloalkyl, heterocyclyl, aryl, heteroaryl, cycloalkoxy, heteroalkoxy, cycloalkylthio, heterocyclylthio and -OR⁴.

The term "haloalkyl" refers to an alkyl group substituted by one or more halogens, wherein the alkyl is as defined above.

The term "haloalkoxy" refers to an -O-(haloalkyl) group, wherein the haloalkyl is as defined above.

The term "deuterated alkyl" refers to an alkyl group substituted by one or more deuterium atoms, wherein the alkyl is as defined above.

The term "deuterated alkoxy" refers to an -O-(deuterated alkyl) group, wherein the deuterated alkyl is as defined above.

The term "heterocyclyloxy" refers to an -O-(heterocyclyl) group, wherein the heterocyclyl is as defined above.

The term "hydroxyalkyl" refers to an alkyl group substituted by hydroxy(s), wherein the alkyl is as defined above.

The term "hydroxy" refers to an -OH group.

The term "halogen" refers to fluorine, chlorine, bromine or iodine.

The term "amino" refers to a -NH₂ group.

The term "cyano" refers to a -CN group.

The term "nitro" refers to a -NO₂ group.

The term "oxo" refers to an =O group.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without paying excessive efforts. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

A "pharmaceutically acceptable salt" refers to a salt of the compound of the present invention, which is safe and effective in mammals and has the desired biological activity.

R⁴ is as defined in the formula (I).

### Synthesis Method of the Compound of the Present Invention

In order to achieve the object of the present invention, the present invention applies the following technical solutions:

### Scheme I

A method for preparing the compound of formula (I) of the present invention or a tautomer, mesomer, racemate, enantiomer, diastereomer thereof, or mixture thereof, or a pharmaceutically acceptable salt thereof, comprises the following steps of: 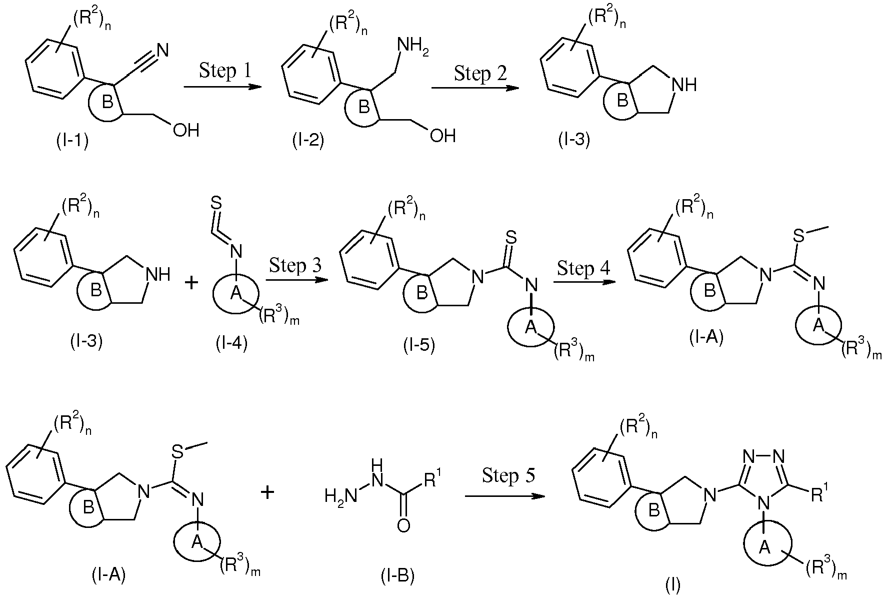
in Step 1, a compound of formula (I-1) is subjected to a reduction reaction in the presence of a reducing reagent to obtain a compound of formula (I-2);
in step 2, the compound of formula (I-2) and thionyl chloride are subjected to a cyclization reaction to obtain a compound of formula (I-3);
in Step 3, the compound of formula (I-3) and a compound of formula (I-4) are heated to obtain a compound of formula (I-5);
in Step 4, the compound of formula (I-5) is reacted with a methylating reagent under an alkaline condition to obtain a compound of formula (I-A);
in Step 5, the compound of formula (I-A) and a compound of formula (I-B) or a hydrochloride salt thereof are subjected to a cyclization reaction under an acidic condition to obtain the compound of formula (I).

The reducing reagent includes, but is not limited to, lithium aluminum hydride, sodium borohydride, DIBAL-H, NaAlH(O-t-Bu)₃, AlH₃, NaCNBH₃, Na(AcO)₃BH, B₂H₅, Li(Et)₃BH, Pd/C/H₂ and Raney Ni/H₂.

The reagent that provides an alkaline condition includes organic bases and inorganic bases. The organic bases include, but are not limited to, triethylamine, *N*,*N*-diisopropylethylamine, *n*-butyllithium, lithium diisopropylamide, lithium bis(trimethylsilyl)amine, potassium acetate, sodium *tert*-butoxide and potassium *tert*-butoxide. The inorganic bases include, but are not limited to, sodium hydride, potassium phosphate, sodium carbonate, potassium carbonate, potassium acetate, cesium carbonate, sodium hydroxide and lithium hydroxide.

The methylating reagent includes, but is not limited to, methyl *p*-toluenesulfonate, methyl iodide, methyl Grignard reagent, dimethyl sulfate, methyl trifluoromethanesulfonate and diazomethane.

The reagent that provides an acidic condition includes, but is not limited to, hydrogen chloride, trifluoroacetic acid, formic acid, acetic acid, hydrochloric acid, sulfuric acid, methanesulfonic acid, nitric acid, phosphoric acid, *p*-toluenesulfonic acid, Me₃SiCl and TMSOT_{f}.

The above reactions are preferably carried out in a solvent. The solvent used includes, but is not limited to, acetic acid, methanol, ethanol, toluene, tetrahydrofuran, dichloromethane, petroleum ether, ethyl acetate, n-hexane, dimethyl sulfoxide, 1,4-dioxane, water, *N*,*N*-dimethylformamide, and mixtures thereof.

Wherein:
ring A, ring B, R¹-R³, n and m are as defined in formula (I).

### Scheme II

A method for preparing the compound of formula (I) of the present invention or a tautomer, mesomer, racemate, enantiomer, diastereomer thereof, or mixture thereof, or a pharmaceutically acceptable salt thereof, comprises the following steps of: 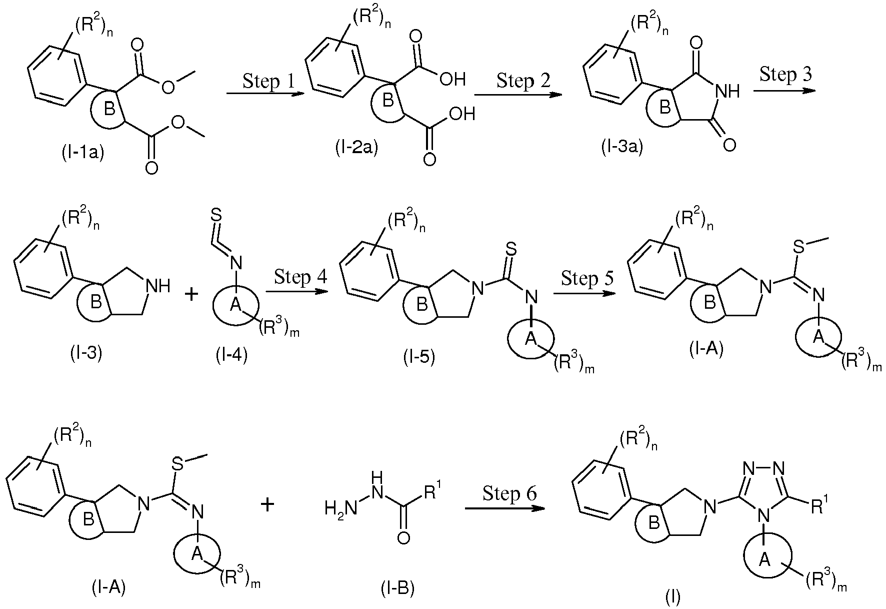
in Step 1, a compound of formula (I-1a) is hydrolyzed under an alkaline condition to obtain a compound of formula (I-2a);
in Step 2, the compound of formula (I-2a) and carbonyl diamine are subjected to a cyclization reaction to obtain a compound of formula (I-3a);
in step 3, the compound of formula (I-3a) is reacted with a reducing reagent to obtain a compound of formula (I-3);
in Step 4, the compound of formula (I-3) and a compound of formula (I-4) are heated to obtain a compound of formula (I-5);
in Step 5, the compound of formula (I-5) is reacted with a methylating reagent under an alkaline condition to obtain a compound of formula (I-A);
in Step 6, the compound of formula (I-A) and a compound of formula (I-B) or a hydrochloride salt thereof are subjected to a cyclization reaction under an acidic condition to obtain the compound of formula (I).

The reducing reagent includes, but is not limited to, lithium aluminum hydride, sodium borohydride, DIBAL-H, NaAlH(O-t-Bu)₃, AlH₃, NaCNBH₃, Na(AcO)₃BH, BH₃ in tetrahydrofuran (IN), B₂H₅, Li(Et)₃BH, Pd/C/H₂ and Raney Ni/H₂.

The reagent that provides an alkaline condition includes organic bases and inorganic bases. The organic bases include, but are not limited to, triethylamine, *N*,*N*-diisopropylethylamine, *n*-butyllithium, lithium diisopropylamide, lithium bis(trimethylsilyl)amine, potassium acetate, sodium *tert*-butoxide and potassium *tert*-butoxide. The inorganic bases include, but are not limited to, sodium hydride, potassium phosphate, sodium carbonate, potassium carbonate, potassium acetate, cesium carbonate, sodium hydroxide and lithium hydroxide.

The methylating reagent includes, but is not limited to, methyl *p*-toluenesulfonate, methyl iodide, methyl Grignard reagent, dimethyl sulfate, methyl trifluoromethanesulfonate and diazomethane.

The reagent that provides an acidic condition includes, but is not limited to, hydrogen chloride, trifluoroacetic acid, formic acid, acetic acid, hydrochloric acid, sulfuric acid, methanesulfonic acid, nitric acid, phosphoric acid, *p*-toluenesulfonic acid, Me₃SiCl and TMSOT_{f}.

The above reactions are preferably carried out in a solvent. The solvent used includes, but is not limited to, acetic acid, methanol, ethanol, toluene, tetrahydrofuran, dichloromethane, petroleum ether, ethyl acetate, *n*-hexane, dimethyl sulfoxide, 1,4-dioxane, water, *N*,*N*-dimethylformamide, and mixtures thereof.

Wherein:
ring A, ring B, R¹-R³, n and m are as defined in formula (I).

### Scheme III

A method for preparing the compound of formula (III) of the present invention or a tautomer, mesomer, racemate, enantiomer, diastereomer thereof, or mixture thereof, or a pharmaceutically acceptable salt thereof, comprises the following steps of: 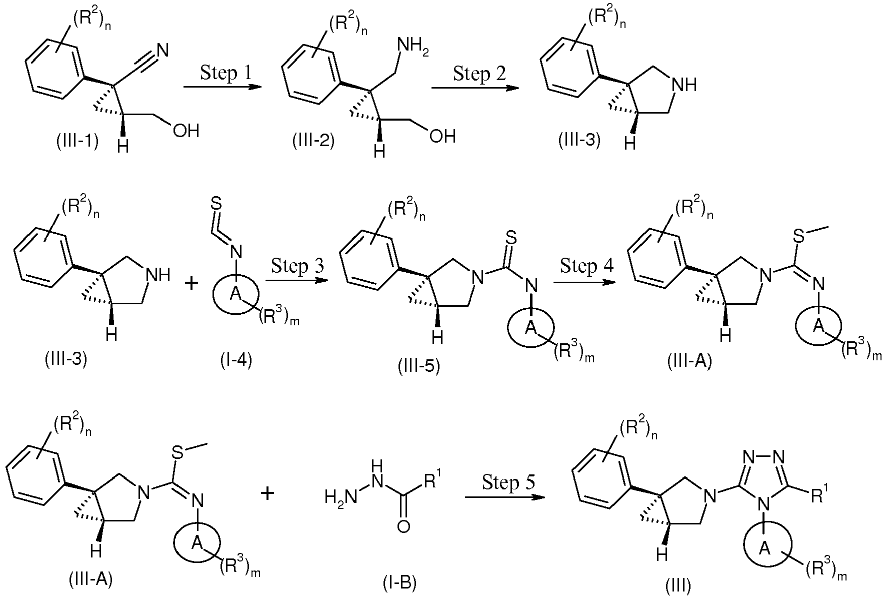
in Step 1, a compound of formula (III-1) is subjected to a reduction reaction in the presence of a reducing reagent to obtain a compound of formula (III-2);
in Step 2, the compound of formula (III-2) and thionyl chloride are subjected to a cyclization reaction to obtain a compound of formula (III-3);
in Step 3, the compound of formula (III-3) and a compound of formula (1-4) are heated to obtain a compound of formula (III-5);
in Step 4, the compound of formula (III-5) is reacted with a methylating reagent under an alkaline condition to obtain a compound of formula (III-A);
in Step 5, the compound of formula (III-A) and a compound of formula (I-B) or a hydrochloride salt thereof are subjected to a cyclization reaction under an acidic condition to obtain the compound of formula (III).

The reducing reagent includes, but is not limited to, lithium aluminum hydride, sodium borohydride, DIBAL-H, NaAlH(O-t-Bu)₃, AlH₃, NaCNBH₃, Na(AcO)₃BH, BH₃ in tetrahydrofuran (IN), B₂H₅, Li(Et)₃BH, Pd/C/H₂ and Raney Ni/H₂.

The reagent that provides an alkaline condition includes organic bases and inorganic bases. The organic bases include, but are not limited to, triethylamine, *N*,*N*-diisopropylethylamine, *n*-butyllithium, lithium diisopropylamide, lithium bis(trimethylsilyl)amine, potassium acetate, sodium *tert*-butoxide and potassium *tert*-butoxide. The inorganic bases include, but are not limited to, sodium hydride, potassium phosphate, sodium carbonate, potassium carbonate, potassium acetate, cesium carbonate, sodium hydroxide and lithium hydroxide.

The methylating reagent includes, but is not limited to, methyl *p*-toluenesulfonate, methyl iodide, methyl Grignard reagent, dimethyl sulfate, methyl trifluoromethanesulfonate and diazomethane.

The reagent that provides an acidic condition includes, but is not limited to, hydrogen chloride, trifluoroacetic acid, formic acid, acetic acid, hydrochloric acid, sulfuric acid, methanesulfonic acid, nitric acid, phosphoric acid, *p*-toluenesulfonic acid, Me₃SiCl, TMSOT_{f}.

The above reactions are preferably carried out in a solvent. The solvent used includes, but is not limited to, acetic acid, methanol, ethanol, toluene, tetrahydrofuran, dichloromethane, petroleum ether, ethyl acetate, *n*-hexane, dimethyl sulfoxide, 1,4-dioxane, water or *N*,*N*-dimethylformamide.

Wherein:
ring A, R¹-R³, n and m are as defined in formula (I).

### PREFERRED EMBODIMENTS

### EXAMPLES

The structures of the compounds were identified by nuclear magnetic resonance (NMR) and/or mass spectrometry (MS). NMR shifts (δ) are given in 10⁻⁶ (ppm). NMR was determined by a Bruker AVANCE-400 machine. The solvents for determination were deuterated-dimethyl sulfoxide (DMSO-*d₆*), deuterated-chloroform (CDCl₃) and deuterated-methanol (CD₃OD), and the internal standard was tetramethylsilane (TMS).

MS was determined by a FINNIGAN LCQAd (ESI) mass spectrometer (manufacturer: Thermo, type: Finnigan LCQ advantage MAX).

High performance liquid chromatography (HPLC) was determined on an Agilent 1200DAD high pressure liquid chromatograph (Sunfire C18 150×4.6 mm chromatographic column), and a Waters 2695-2996 high pressure liquid chromatography spectrometer (Gimini C18 150×4.6 mm chromatographic column).

Chiral HPLC was determined on a LC-10A vp (Shimadzu) or SFC-analytical (Berger Instruments Inc.).

Yantai Huanghai HSGF254 or Qingdao GF254 silica gel plate was used as the thin-layer silica gel chromatography (TLC) plate. The dimension of the silica gel plate used in TLC was 0.15 mm to 0.2 mm, and the dimension of the silica gel plate used in product purification was 0.4 mm to 0.5 mm.

Yantai Huanghai 200 to 300 mesh silica gel was generally used as a carrier for column chromatography.

Prep Star SD-1 (Varian Instruments Inc.) or SFC-multigram (Berger Instruments Inc.) was used for chiral preparative column chromatography.

CombiFlash rapid preparation instrument used was Combiflash Rf200 (TELEDYNE ISCO).

The average kinase inhibition rates and IC₅₀ values were determined by a NovoStar ELISA (BMG Co., Germany).

The known starting materials of the present invention can be prepared by the known methods in the art, or can be purchased from ABCR GmbH & Co. KG, Acros Organnics, Aldrich Chemical Company, Accela ChemBio Inc., or Dari chemical Company, etc.

Unless otherwise stated, the reactions were carried out under argon atmosphere or nitrogen atmosphere.

"Argon atmosphere" or "nitrogen atmosphere" means that a reaction flask is equipped with an argon or nitrogen balloon (about1 L).

"Hydrogen atmosphere" means that a reaction flask is equipped with a hydrogen balloon (about1 L).

Pressurized hydrogenation reactions were performed on a Parr 3916EKX hydrogenation instrument and a Qinglan QL-500 hydrogen generator or HC2-SS hydrogenation instrument.

In hydrogenation reactions, the reaction system was generally vacuumed and filled with hydrogen, with the above operation was repeated three times.

CEM Discover-S 908860 type microwave reactor was used in microwave reactions.

Unless otherwise stated, the solution refers to an aqueous solution.

Unless otherwise stated, the reaction temperature is room temperature from 20°C to 30°C.

The reaction process in the examples was monitored by thin layer chromatography (TLC). The developing solvent used in the reactions, the eluent system in column chromatography and the developing solvent system in thin layer chromatography for purification of the compounds included: A: dichloromethane/methanol system, B: *n*-hexane/ethyl acetate system, and C: petroleum ether/ethyl acetate system. The ratio of the volume of the solvent was adjusted according to the polarity of the compounds, and a small quantity of alkaline reagent such as triethylamine or acidic reagent such as acetic acid can also be added for adjustment.

### Example 1

### (1S,SR)-1-(2-Chloro-4-fluorophenyl)-3-(5-((2-fluoroethoxy)methyl)-4-(6-methoxypyrid in-3-yl)-4H-1,2,4-triazol-3-yl)-3-azabicyclo[3.1.0]hexane

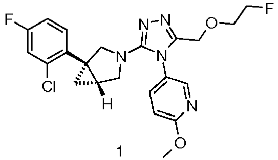

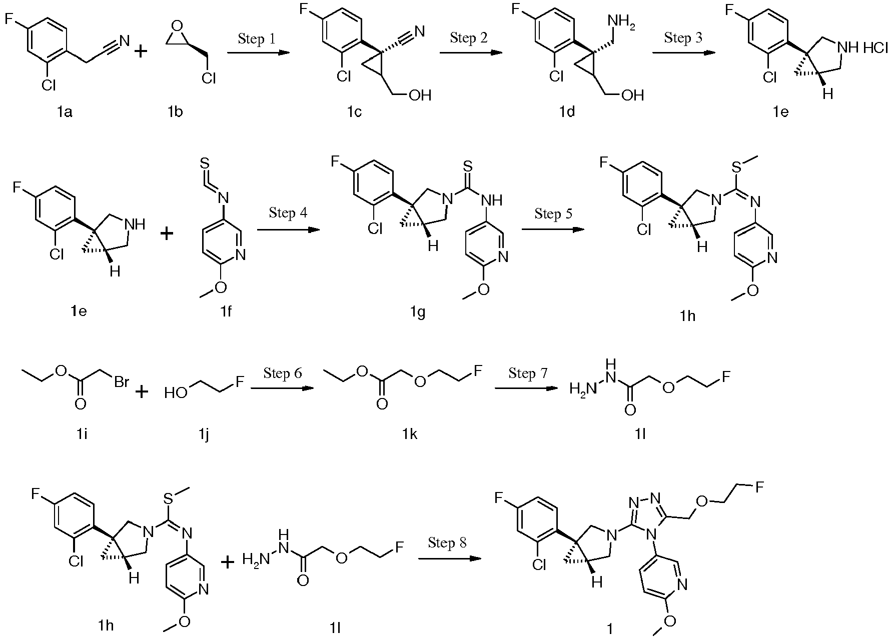

### Step1

### (1S)-1-(2-Chloro-4-fluorophenyl)-2-(hydroxymethyl)cyclopropanecarbonitrile 1c

2-Chloro-4-fluorophenylacetonitrile **1a** (1 g, 5.9 mmol) was dissolved in 20 mL of tetrahydrofuran. The reaction solution was cooled to -20°C in a dry ice-acetone bath, and added slowly with sodium bis(trimethylsilyl)amide (5.9 mL, 11.8 mmol). After completion of the addition, the reaction solution was stirred for 30 minutes, and then added with (*R*)-2-(chloromethyl)oxirane **1b** (600 mg, 6.49 mmol). After completion of the addition, the dry ice-acetone bath was removed. The reaction solution was naturally warmed up to room temperature, and stirred for 2 hours. The reaction was quenched with saturated ammonium chloride solution (20 mL), and the reaction solution was extracted with ethyl acetate (50 mL×3). The organic phases were combined, washed with saturated sodium chloride solution (50 mL×3), and concentrated under reduced pressure to obtain the crude title product **1c** (1.35 g), which was used directly in the next step without purification.
MS m/z (ESI): 226.4 [M+1].

### Step 2

### ((2S)-2-(Aminomethyl)-2-(2-chloro-4-fluorophenyl)cyclopropyl)methanol 1d

Lithium aluminum hydride (672 mg, 17.7 mmol) was added to 15 mL of tetrahydrofuran. The reaction solution was cooled in an ice bath, and added with the crude product **1c** (1.33 g, 5.9 mmol). After completion of the addition, the ice bath was removed. The reaction solution was naturally warmed up to room temperature, and stirred for 15 hours. The reaction solution was added with water (0.7 mL), sodium hydroxide solution (10%, 0.7 mL) and water (2.1 mL) successively, and stirred for 30 minutes after completion of the addition. The reaction solution was filtrated through celite, and the filtrate was concentrated under reduced pressure to obtain the crude title product **1d** (1.4 g), which was used directly in the next step without purification.
MS m/z (ESI): 230.3 [M+1].

### Step 3

### (1S,5R)-1-(2-Chloro-4-fluorophenyl)-3-azabicyclo[3.1.0]hexane hydrochloride 1e

The crude product **1d** (1.35 g, 5.9 mmol) and thionyl chloride (1.05 g, 8.85 mmol) were added to 10 mL of dichloromethane. After completion of the addition, the reaction solution was stirred for 3 hours. The reaction solution was concentrated under reduced pressure to obtain the crude title product **1e** (1.3 g), which was used directly in the next step without purification.
MS m/z (ESI): 212.3 [M+1].

### Step 4

### (1S,5R)-1-(2-Chloro-4-fluorophenyl)-N-(6-methoxypyridin-3-yl)-3-azabicyclo[3.1.0]he xane-3-carbothioamide 1g

5-Isothiocyanato-2-methoxypyridine **1f** (1.25 g, 7.5 mmol, prepared according to the known method disclosed in "Bioorganic and Medicinal Chemistry Letters, 2010, 20(2), 516 - 520") and the crude product **1e** (1.06 g, 5.0 mmol) were added to 20 mL of tetrahydrofuran. After completion of the addition, the reaction solution was stirred for 2 hours. The reaction solution was concentrated under reduced pressure to obtain the crude title product **1g** (1.9 g), which was used directly in the next step without purification.
MS m/z (ESI): 378.2 [M+1].

### Step 5

### Methyl (1S,5R,E)-1-(2-chloro-4-fluorophenyl)-N-(6-methoxypyridin-3-yl)-3-azabicyclo[3.1.0]hexane-3-carbimidothioate 1h

The crude product **1g** (1.86 g, 5.0 mmol) was added to 30 mL of tetrahydrofuran. The reaction solution was cooled in an ice bath, and added with potassium *tert*-butoxide (2.2 g, 20 mmol). After completion of the addition, the reaction solution was stirred for 2 hours, and then added with methyl *p*-toluenesulfonate (1.86 g, 10.0 mmol). After completion of the addition, the ice bath was removed. The reaction solution was naturally warmed up to room temperature, and stirred for 15 hours. The reaction solution was added with ice water (90 mL), and then extracted with ethyl acetate (50 mL×3). The organic phases were combined, and concentrated under reduced pressure. The resulting residue was purified by silica gel column chromatography with elution system B to obtain the title product **1h** (700 mg), yield: 32.2%.
MS m/z (ESI): 392.2 [M+1].

### Step 6

### Ethyl 2-(2-fluoroethoxy)acetate 1k

2-Fluoroethanol (800 mg, 12.49 mmol) and sodium hydride (1.74 g, 10.42 mmol) were added to 30 mL of tetrahydrofuran. The reaction solution was stirred for 2 hours, and then added with ethyl 2-bromoacetate **1i** (1.74 g, 10.42 mmol). After completion of the addition, the reaction solution was stirred for 15 hours. The reaction was quenched with 30 mL of water, and the reaction solution was extracted with ethyl acetate (50 mL×3). The organic phases were combined, washed with saturated sodium chloride solution (50 mL×3), and concentrated under reduced pressure to obtain the crude title product **1k** (300 mg), which was used directly in the next step without purification.
MS m/z (ESI): 151.2 [M+1].

### Step 7

### 2-(2-Fluoroethoxy)acetohydrazide 1l

The crude product **1k** (250 mg, 1.67 mmol) and hydrazine hydrate (85%, 213 mg) were added to 3 mL of ethanol. The reaction solution was added to a sealed tube, and stirred for 15 hours at 80°C. After stopping heating, the reaction solution was concentrated under reduced pressure to obtain the crude title product **1l** (250 mg), which was used directly in the next step without purification.
MS m/z (ESI):137.2 [M+1].

### Step 8

### (1S,5R)-1-(2-Chloro-4-fluorophenyl)-3-(5-((2-fluoroethoxy)methyl)-4-(6-methoxypyrid in-3-yl)-4H-1,2,4-triazol-3-yl)-3-azabicyclo[3.1.0]hexane 1

**1h** (30 mg, 0.08 mmol), the crude product **1l** (31 mg, 0.23 mmol) and trifluoroacetic acid (9 mg, 0.08 mmol) were added to 5 mL of tetrahydrofuran. After completion of the addition, the reaction solution was heated to 65°C and stirred for 1 hour. After stopping heating, the reaction solution was concentrated under reduced pressure. The resulting residue was purified by thin layer chromatography with developing solvent system A to obtain the title product **1** (10 mg), yield: 26.4%.
MS m/z (ESI): 462.1 [M+1].
¹H NMR (400 MHz, CDCl₃): *δ* 8.17 (s, 1H), 7.59 (d, 1H), 7.27 (d, 1H), 7.09 (d, 1H), 6.87-6.85 (m, 2H), 4.53 (d, 1H), 4.41 (s, 3H), 3.99 (s, 3H), 3.64-3.62 (m, 4H), 3.42-3.40 (m, 2H), 1.73-1.71 (m, 1H), 0.98-0.95 (m,2H).

### Example 2

### (1S,5R)-1-(2-Chloro-4-fluorophenyl)-3-(4-(6-methoxypyridin-3-yl)-5-(((tetrahydro-2H-pyran-4-yl)oxy)methyl)-4H-1,2,4-triazol-3-yl)-3-azabicyclo[3.1.0]hexane

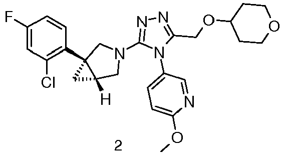

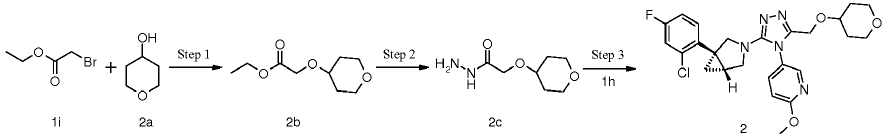

### Step 1

### Ethyl 2-((tetrahydro-2H-pyran-4-yl)oxy)acetate 2b

Tetrahydropyran-4-ol **2a** (1.0 g, 9.8 mmol) was added to 150 mL of tetrahydrofuran. The reaction solution was cooled in an ice bath, and added with **1i** (1.96 g, 11.8 mmol). After completion of the addition, the reaction solution was stirred for 30 minutes, and then added with sodium hydride (352 mg, 14.7 mmol). The ice bath was removed, and the reaction solution was stirred for 6 hours. The reaction solution was added with 30 mL of ice water, and then extracted with ethyl acetate (30 mL×3). The organic phases were combined, washed with saturated sodium chloride solution (50 mL×3), dried over anhydrous sodium sulfate and filtrated to remove the drying agent. The filtrate was concentrated under reduced pressure to obtain the title product **2b** (1.8 g), yield: 87.9%.
MS m/z (ESI): 189.2 [M+1].

### Step 2

### 2-((Tetrahydro-2H-pyran-4-yl)oxy)acetohydrazide 2c

**2b** (1.8 g, 9.6 mmol) and hydrazine hydrate (478 mg, 9.6 mmol) were added to 5 mL of ethanol. The reaction solution was added to a sealed tube, and stirred for 48 hours at 80°C. After stopping heating, the reaction solution was concentrated under reduced pressure to obtain the crude title product **2c** (1.6 g), which was used directly in the next step without purification.
MS m/z (ESI): 175.0 [M+1].

### Step 3

### (1S,5R)-1-(2-Chloro-4-fluorophenyl)-3-(4-(6-methoxypyridin-3-yl)-5-(((tetrahydro-2H-pyran-4-yl)oxy)methyl)-4H-1,2,4-triazol-3-yl)-3-azabicyclo[3.1.0]hexane 2

**1h** (40 mg, 0.1 mmol), the crude product **2c** (53 mg, 0.31 mmol) and trifluoroacetic acid (1 mg, 0.01 mmol) were added to 10 mL of tetrahydrofuran. After completion of the addition, the reaction solution was heated to 70°C and stirred for 3 hours. After stopping heating, the reaction solution was concentrated under reduced pressure. The resulting residue was purified by high performance liquid chromatography to obtain the title product **2** (10 mg), yield: 18.2%.
MS m/z (ESI): 500.1 [M+1].
¹H NMR (400 MHz, CDCl₃): *δ* 8.19-8.18 (d, 1H), 7.57-7.54 (m, 1H), 7.29-7.25 (m, 1H), 7.10-7.08 (m, 1H), 6.88-6.86 (m, 2H), 4.38 (s, 2H), 4.01 (s, 3H), 3.83-3.75 (m, 2H), 3.69-3.61 (m, 1H), 3.54-3.45 (m, 1H), 3.44-3.30 (m, 5H), 1.82-1.73 (m, 2H), 1.48-1.43 (m, 2H), 0.99-0.97 (m,3H).

### Example 3

### (1S,5R)-1-(2-Chloro-4-fluorophenyl)-3-(4-(6-methoxypyridin-3-yl)-5-((((S)-tetrahydrof uran-3-yl)oxy)methyl)-4H-1,2,4-triazol-3-yl)-3-azabicyclo[3.1.0]hexane

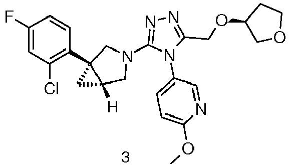

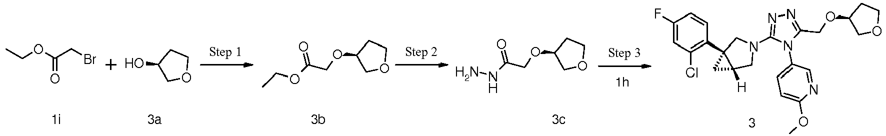

### Step 1

### Ethyl (S)-2-((tetrahydrofuran-3-yl)oxy)acetate 3b

(*S*)-3-Hydroxytetrahydrofuran **3a** (4 g, 45.4 mmol) was added to 150 mL of tetrahydrofuran. The reaction solution was cooled in an ice bath, added with sodium hydride (2.72 g, 68.1 mmol), stirred for 30 minutes, and then added with **1i** (7.58 g, 45.4 mmol). After completion of the addition, the ice bath was removed, and the reaction solution was stirred for 6 hours. The reaction solution was added with 100 mL of ice water, and then extracted with ethyl acetate (30 mL×3). The organic phases were combined, washed with saturated sodium chloride solution (50 mL×3), dried over anhydrous sodium sulfate and filtrated to remove the drying agent. The filtrate was concentrated under reduced pressure to obtain the crude title product **3b** (4.5 g), which was used directly in the next step without purification.
MS m/z (ESI): 175.2 [M+1].

### Step 2

### (S)-2-((Tetrahydrofuran-3-yl)oxy)acetohydrazide 3c

The crude product **3b** (1 g, 5.7 mmol) and hydrazine hydrate (287 mg, 5.7 mmol) were added to 5 mL of ethanol. The reaction solution was added to a sealed tube, and stirred for 18 hours at 80°C. After stopping heating, the reaction solution was concentrated under reduced pressure to obtain the crude title product **3c** (1.1 g), which was used directly in the next step without purification.
MS m/z (ESI):161.2 [M+1].

### Step 3

### (1S,5R)-1-(2-Chloro-4-fluorophenyl)-3-(4-(6-methoxypyridin-3-yl)-5-((((S)-tetrahydrof uran-3-yl)oxy)methyl)-4H-1,2,4-triazol-3-yl)-3-azabicyclo[3.1.0]hexane 3

**1h** (50 mg, 0.13 mmol), the crude product **3c** (31 mg, 0.19 mmol) and trifluoroacetic acid (1 mg, 0.01 mmol) were added to 20 mL of tetrahydrofuran. After completion of the addition, the reaction solution was heated to 70°C and stirred for 3 hours. After stopping heating, the reaction solution was concentrated under reduced pressure. The resulting residue was purified by high performance liquid chromatography to obtain the title product **3** (15 mg), yield: 24.2%.
MS m/z (ESI): 486.2 [M+1].
¹H NMR (400 MHz, DMSO-d*₆*): *δ* 8.20 (s, 1H), 7.60 (d, 1H), 7.31-7.29 (m, 1H), 7.14 (d, 1H), 6.91-6.89 (m, 2H), 4.37 (s, 2H), 4.18-4.17 (m, 1H), 4.05 (s, 3H), 3.81-3.67 (m, 5H), 3.50 (d, 2H), 3.41-3.38 (m, 1H), 1.77-1.63 (m, 3H), 1.02-1.00 (m, 2H).

### Example 4

### (1S,5R)-1-(2-Chloro-3-fluorophenyl)-3-(5-(methoxymethyl)-4-(6-methoxypyridin-3-yl)-4H-1,2,4-triazol-3-yl)-3-azabicyclo[3.1.0]hexane

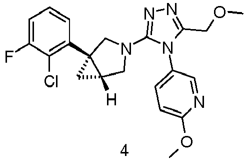

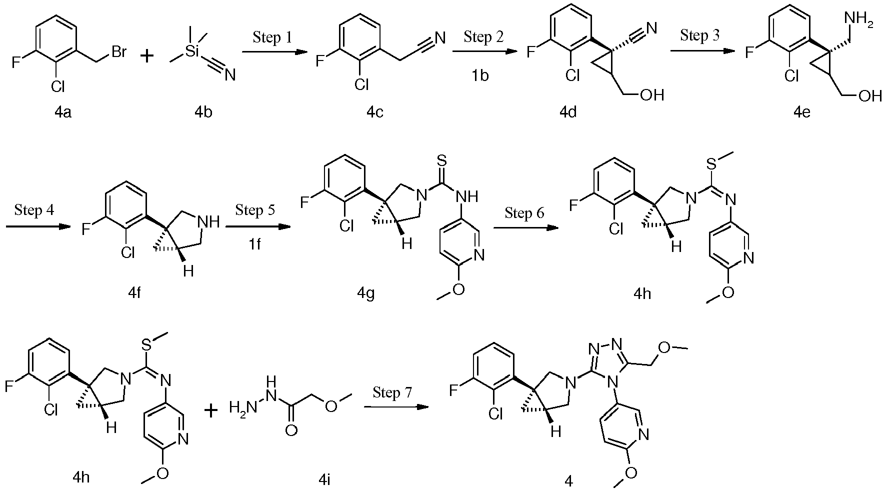

### Step 1

### 2-(2-Chloro-3-fluorophenyl)acetonitrile 4c

2-Chloro-3-fluorobenzyl bromide **4a** (1.0 g, 4.47 mmol) was added to 10 mL of acetonitrile. The reaction solution was cooled in an ice bath, and added with trimethylcyanosilane **4b** (532 mg, 5.37 mmol). After completion of the addition, the ice bath was removed. The reaction solution was naturally warmed up to room temperature, and stirred for 15 hours. The reaction solution was concentrated under reduced pressure, and the resulting residue was purified by silica gel column chromatography with elution system B to obtain the title product **4c** (500 mg), yield: 65.9%.
MS m/z (ESI): 170.2 [M+1].

### Step 2

### (1S)-1-(2-Chloro-3 -fluorophenyl)-2-(hydroxymethyl)cyclopropanecarbonitrile 4d

**4c** (500 mg, 2.95 mmol) was added to 10 mL of tetrahydrofuran. The reaction solution was cooled in an ice bath, and added slowly with sodium bis(trimethylsilyl)amide (1.1 g, 5.9 mmol). After completion of the addition, the reaction solution was stirred for 1 hour, and then added with **1b** (273 mg, 2.95 mmol). After completion of the addition, the ice bath was removed. The reaction solution was naturally warmed up to room temperature, and stirred for 2 hours. The reaction was quenched with saturated ammonium chloride solution (20 mL), and the reaction solution was extracted with ethyl acetate (50 mL×3). The organic phases were combined, washed with saturated sodium chloride solution (50 mL×3), and concentrated under reduced pressure to obtain the crude title product **4d** (670 mg), which was used directly in the next step without purification.
MS m/z (ESI): 226.2 [M+1].

### Step 3

### ((2S)-2-(Aminomethyl)-2-(2-chloro-3-fluorophenyl)cyclopropyl)methanol 4e

Lithium aluminum hydride (336 mg, 8.85 mmol) was added to 10 mL of tetrahydrofuran. The reaction solution was cooled in an ice bath, and added with the crude product **4d** (666 mg, 2.95 mmol). After completion of the addition, the ice bath was removed. The reaction solution was naturally warmed up to room temperature, and stirred for 15 hours. The reaction solution was added with water (0.35 mL), sodium hydroxide solution (10%, 0.35 mL) and water (1 mL) successively, and stirred for 30 minutes after completion of the addition. The reaction solution was filtrated through celite, and the filtrate was concentrated under reduced pressure to obtain the crude title product **4e** (700 mg), which was used directly in the next step without purification.
MS m/z (ESI): 230.3 [M+1].

### Step 4

### (1S,5R)-1-(2-Chloro-3-fluorophenyl)-3-azabicyclo[3.1.0]hexane 4f

The crude product **4e** (678 mg, 2.95 mmol) and thionyl chloride (526 mg, 4.43 mmol) were added to 10 mL of dichloromethane. After completion of the addition, the reaction solution was stirred for 3 hours. The reaction solution was concentrated under reduced pressure to obtain the crude title product **4f** (600 mg), which was used directly in the next step without purification.
MS m/z (ESI): 212.2 [M+1].

### Step 5

### (1S,5R)-1-(2-Chloro-3-fluorophenyl)-N-(6-methoxypyridin-3-yl)-3-azabicyclo[3.1.0]he xane-3-carbothioamide 4g

The crude product **4f** (212 mg, 1 mmol) and **1f** (332 mg, 2 mmol) were added to 10 mL of tetrahydrofuran. After completion of the addition, the reaction solution was stirred for 2 hours. The reaction solution was concentrated under reduced pressure to obtain the crude title product **4g** (350 mg), which was used directly in the next step without purification.
MS m/z (ESI): 378.3 [M+1].

### Step 6

### Methyl (1S,5R,E)-1-(2-chloro-3-fluorophenyl)-N-(6-methoxypyridin-3-yl)-3-azabicyclo[3.1.0]hexane-3-carbimidothioate 4h

The crude product **4g** (378 mg, 1 mmol) was added to 10 mL of tetrahydrofuran. The reaction solution was cooled in an ice bath, and added with potassium *tert*-butoxide (337 mg, 3 mmol). After completion of the addition, the reaction solution was stirred for 1 hour, and then added with methyl *p*-toluenesulfonate (372 mg, 2 mmol). After completion of the addition, the ice bath was removed. The reaction solution was naturally warmed up to room temperature, and stirred for 15 hours. The reaction solution was added with ice water (30 mL), and then extracted with ethyl acetate (50 mL×3). The organic phases were combined, washed with saturated sodium chloride solution (50 mL×3), and concentrated under reduced pressure. The resulting residue was purified by silica gel column chromatography with elution system B to obtain the title product **4h** (200 mg), yield: 45.9%.
MS m/z (ESI): 392.3 [M+1].

### Step 7

### (1S,5R)-1-(2-Chloro-3-fluorophenyl)-3-(5-(methoxymethyl)-4-(6-methoxypyridin-3-yl)-4H-1,2,4-triazol-3-yl)-3-azabicyclo[3.1.0]hexane 4

**4h** (392 mg, 1 mmol), methoxyacetohydrazide **4i** (521 mg, 5 mmol) and trifluoroacetic acid (114 mg, 1 mmol) were added to 5 mL of tetrahydrofuran. After completion of the addition, the reaction solution was heated to 70°C and stirred for 3 hours. After stopping heating, the reaction solution was concentrated under reduced pressure. The resulting residue was purified by thin layer chromatography with developing solvent system A to obtain the title product **4** (30 mg), yield: 6.5%.
MS m/z (ESI):430.2 [M+1].
¹H NMR (400 MHz, CDCl₃): *δ* 8.16 (s, 1H), 7.56 (d, 1H), 7.27 (d, 1H), 7.14 (d, 2H), 6.85 (d, 1H), 4.27 (s, 2H), 3.99 (s, 3H), 3.65 (d, 1H), 3.47 (d, 2H), 3.36 (d, 1H), 3.27 (s, 3H), 1.77-1.75 (m, 1H), 1.01-0.99 (m, 2H).

### Example 5

### (1S,5R)-3-(4-(Benzo[d][1,3]dioxol-5-yl)-5-(methoxymethyl)-4H-1,2,4-triazol-3-yl)-1-(2 -chloro-4-fluorophenyl)-3-azabicyclo[3.1.0]hexane

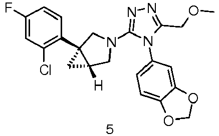

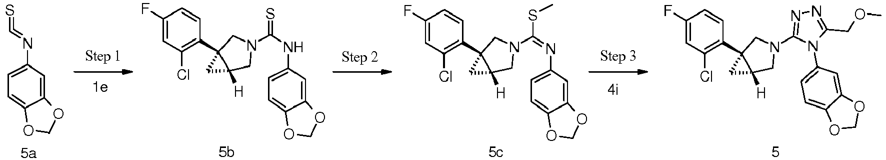

### Step 1

### (1S,5R)-N-(Benzo[d][1,3]dioxol-5-yl)-1-(2-chloro-4-fluorophenyl)-3-azabicyclo[3.1.0]h exane-3-carbothioamide 5b

Benzo[*d*][1,3]dioxole-5-yl isothiocyanate **5a** (311 mg, 1.74 mmol, prepared according to the known method disclosed in "Journal of Medicinal Chemistry, 2015, 58(3), 1123-1139") and the crude product **1e** (245 mg, 1.16 mmol) were added to 10 mL of tetrahydrofuran. After completion of the addition, the reaction solution was stirred for 2 hours. The reaction solution was concentrated under reduced pressure to obtain the crude title product **5b** (450 mg), which was used directly in the next step without purification.
MS m/z (ESI): 391.2 [M+1].

### Step 2

### Methyl (1S,5R,E)-N-benzo[d][1,3]dioxol-5-yl-1-(2-chloro-4-fluorophenyl)-3-azabicyclo[3.1.0]hexane-3-carbimidothioate 5c

The crude product **5b** (391 mg, 1 mmol) was added to 30 mL of tetrahydrofuran. The reaction solution was cooled in an ice bath, and added with potassium *tert*-butoxide (337 mg, 3 mmol). After completion of the addition, the reaction solution was stirred for 2 hour, and then added with methyl p-toluenesulfonate (372 mg, 2 mmol). After completion of the addition, the ice bath was removed. The reaction solution was naturally warmed up to room temperature, and stirred for 15 hours. The reaction solution was added with ice water (80 mL), and then extracted with ethyl acetate (50 mL×3). The organic phases were combined, washed with saturated sodium chloride solution (50 mL×3), and concentrated under reduced pressure. The resulting residue was purified by thin layer chromatography with developing solvent system A to obtain the title product **5c** (150 mg), yield: 33.3%.
MS m/z (ESI): 405.3 [M+1].

### Step 3

### (1S,5R)-3-(4-(Benzo[d][1,3]dioxol-5-yl)-5-(methoxymethyl)-4H-1,2,4-triazol-3-yl)-1-(2 -chloro-4-fluorophenyl)-3-azabicyclo[3.1.0]hexane 5

**5c** (80 mg, 0.2 mmol), **4i** (103 mg, 0.99 mmol) and trifluoroacetic acid (23 mg, 0.2 mmol) were added to 5 mL of tetrahydrofuran. After completion of the addition, the reaction solution was heated to 70°C and stirred for 3 hours. After stopping heating, the reaction solution was concentrated under reduced pressure. The resulting residue was purified by thin layer chromatography with developing solvent system A to obtain the title product 5 (20 mg), yield: 21.7%.
MS m/z (ESI): 443.3 [M+1].
¹H NMR (400 MHz, CDCl₃): *δ* 7.27 (t, 1H), 7.09 (d, 1H), 6.88 (d, 2H), 6.81 (d, 2H), 6.10 (s, 2H), 4.28 (s, 2H), 3.65 (d, 1H), 3.47 (d, 2H), 3.34 (d, 1H), 3.32 (s, 3H), 1.72-1.69 (m, 1H), 1.01-0.99 (m, 2H).

### Example 6

### (1S,5R)-1-(2-Chloro-4-fluorophenyl)-3-(5-(ethoxymethyl)-4-(6-methoxypyridin-3-yl)-4 H-1,2,4-triazol-3-yl)-3-azabicyclo[3.1.0]hexane 6

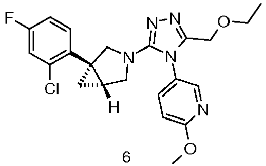

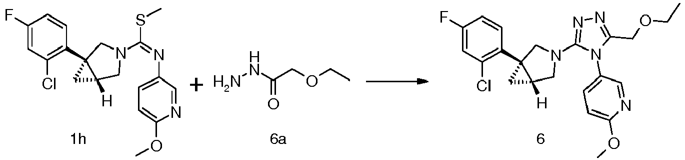

**1h** (40 mg, 0.1 mmol), 2-ethoxyacetohydrazide **6a** (60 mg, 0.51 mmol) and trifluoroacetic acid (12 mg, 0.1 mmol) were added to 5 mL of tetrahydrofuran. After completion of the addition, the reaction solution was heated to 70°C and stirred for 3 hours. After stopping heating, the reaction solution was concentrated under reduced pressure. The resulting residue was purified by thin layer chromatography with developing solvent system A to obtain the title product **6** (10 mg), yield: 20.3%.
MS m/z (ESI): 444.2 [M+1].
¹H NMR (400 MHz, CDCl₃): *δ* 8.15 (s, 1H), 7.56 (d, 1H), 7.27 (d, 1H), 7.08 (d, 1H), 7.05 (d, 1H), 6.86 (d, 1H), 4.31 (s, 2H), 3.99 (s, 3H), 3.62-3.60 (m, 1H), 3.45-3.42 (m, 4H), 3.35 (d, 1H), 1.72-1.70 (m, 1H), 1.09 (t, 3H), 0.97-0.95 (m, 2H).

### Example 7

### (1S,SR)-1-(2-Chloro-4-fluorophenyl)-3-(4-(6-methoxypyridin-3-yl)-5-methyl-4H-1,2,4-t riazol-3-yl)-3-azabicyclo[3.1.0]hexane 7

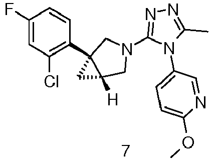

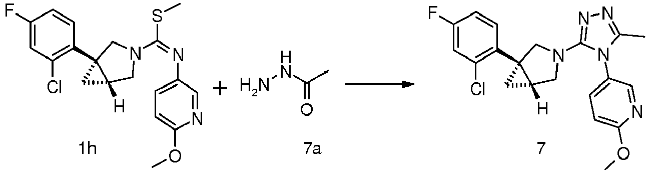

**1h** (80 mg, 0.2 mmol), acetohydrazide **7a** (76 mg, 1.02 mmol) and trifluoroacetic acid (23 mg, 0.2 mmol) were added to 5 mL of tetrahydrofuran. After completion of the addition, the reaction solution was heated to 70°C and stirred for 3 hours. After stopping heating, the reaction solution was concentrated under reduced pressure. The resulting residue was purified by thin layer chromatography with developing solvent system A to obtain the title product **7** (25 mg), yield: 28.3%.
MS m/z (ESI): 400.2 [M+1].
¹H NMR (400 MHz, CDCl₃): *δ* 8.08 (s, 1H), 7.46 (d, 1H), 7.27 (d, 1H), 7.07 (d, 1H), 688-6.86 (m, 2H), 4.00 (s, 3H), 3.64 (d, 1H), 3.36-3.34 (m, 3H), 2.15 (s, 3H), 1.71-1.69 (m, 1H), 0.99-0.96 (m, 2H).

### Example 8

### 1-(2-Chloro-4-fluorophenyl)-3-(5-(methoxymethyl)-4-(6-methoxypyridin-3-yl)-4H-1,2, 4-triazol-3-yl)-3-azabicyclo[3.1.0]hexane 8

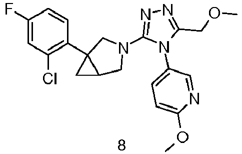

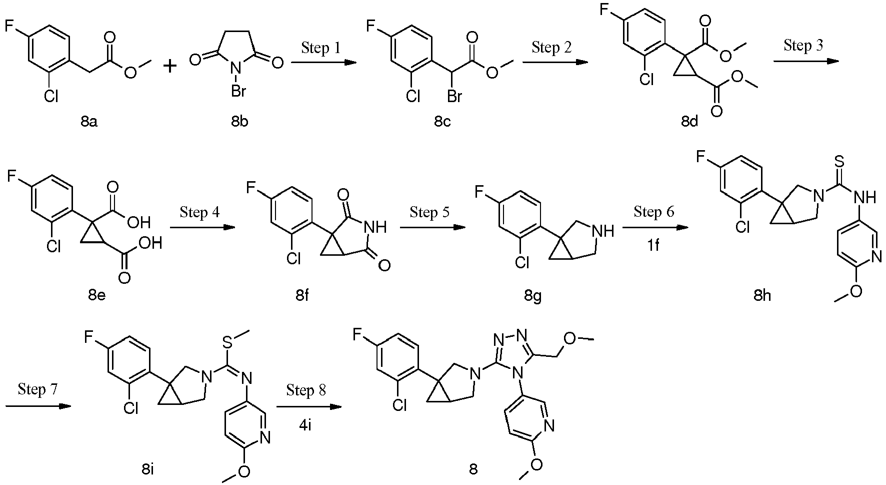

### Step 1

### Methyl 2-bromo-2-(2-chloro-4-fluorophenyl)acetate 8c

Methyl 2-(2-chloro-4-fluorophenylacetate **8a** (2.4 g, 11.8 mmol), *N*-bromosuccinimide **8b** (2.4 g, 13.5 mmol) and hydrobromic acid (40%, one drop) were added to 25 mL of carbon tetrachloride. After completion of the addition, the reaction solution was heated to 78°C and stirred for 18 hours. After stopping heating, the reaction solution was naturally cooled to room temperature and filtrated. The filtrate was concentrated under reduced pressure to obtain the crude title product **8c** (4.3 g), which was used directly in the next step without purification.
MS m/z (ESI): 280.3 [M+1].

### Step 2

### Dimethyl 1-(2-chloro-4-fluorophenyl)cyclopropane-1,2-dicarboxylate 8d

The crude product **8c** (4.3 g, 15 mmol) and methyl acrylate (2 mL, 22 mmol) were added to 20.4 mL of a mixed solvent of diethyl ether and ethanol (V:V = 50:1), the resulting solution was then added to a solution obtained by adding sodium hydride (720 mg, 18 mmol) to 50.5 mL of a mixed solvent of diethyl ether and ethanol (V:V = 100:1). The reaction solution was stirred for 23 hours. The reaction was quenched with 80 mL of water, and the reaction solution was extracted with ethyl acetate (50 mL×3). The organic phases were combined, and concentrated under reduced pressure to obtain the crude title product **8d** (4 g), which was used directly in the next step without purification.
MS m/z (ESI): 287.3 [M+1].

### Step 3

### 1-(2-Chloro-4-fluorophenyl)cyclopropane-1,2-dicarboxylic acid 8e

The crude product **8d** (4 g, 14 mmol) was added to 60 mL of a mixed solvent of ethanol and water (V: V = 1:1), followed by addition of potassium hydroxide (3 g, 53.5 mmol). After completion of the addition, the reaction solution was heated to 65°C and stirred for 15 hours. After stopping heating, the reaction solution was naturally cooled to room temperature and extracted with ethyl acetate (50 mL×3). The aqueous phase was added dropwise with 2 N hydrochloric acid to adjust the pH to pH 2-3, and then extracted with ethyl acetate (50 mL×3). The organic phases were combined, and concentrated under reduced pressure to obtain the crude title product **8e** (1.9 g), which was used directly in the next step without purification.
MS m/z (ESI): 257.2 [M-1].

### Step 4

### 1-(2-Chloro-4-fluorophenyl)-3-azabicyclo[3.1.0]hexane-2,4-dione 8f

The crude product **8e** (1.9 g, 7.5 mmol) and carbonyl diamine (1.35 g, 22.5 mmol) were added to 20 mL of 1,4-xylene. After completion of the addition, the reaction solution was heated to 120°C and stirred for 18 hours. After stopping heating, the reaction solution was concentrated under reduced pressure. The resulting residue was purified by high performance liquid chromatography to obtain the title product **8f** (110 mg), yield: 6.1%.
MS m/z (ESI): 240.2 [M+1].

### Step 5

### 1-(2-Chloro-4-fluorophenyl)-3-azabicyclo[3.1.0]hexane 8g

**8f** (100 mg, 0.37 mmol) and borane in tetrahydrofuran (1 N, 2 mL) were added to 5 mL of tetrahydrofuran. After completion of the addition, the reaction solution was stirred for 15 hours. The reaction solution was then heated to 60°C, and stirred for 1 hour. The reaction solution was added with hydrochloric acid (6 N, 2 mL) and stirred for 15 minutes. After stopping heating, the reaction solution was evaporated under reduced pressure to remove tetrahydrofuran, and added dropwise with 5 N sodium hydroxide solution to adjust the pH to 12. The reaction solution was stirred for 15 minutes, and extracted with ethyl acetate (20 mL×3). The organic phases were combined, washed with saturated sodium chloride solution (15 mL×2), dried over anhydrous sodium sulfate and filtrated to remove the drying agent. The filtrate was concentrated under reduced pressure to obtain the crude title product **8g** (90 mg), which was used directly in the next step without purification.
MS m/z (ESI): 212.2 [M+1].

### Step 6

### 1-(2-Chloro-4-fluorophenyl)-N-(6-methoxypyridin-3-yl)-3-azabicyclo[3.1.0]hexane-3-c arbothioamide 8h

The crude product **8g** (90 mg, 0.37 mmol) and **1f** (90 mg, 0.55 mmol) were added to 5 mL of tetrahydrofuran. After completion of the addition, the reaction solution was heated to 50°C and stirred for 18 hours. The reaction solution was concentrated under reduced pressure to obtain the crude title product **8h** (140 mg), which was used directly in the next step without purification.
MS m/z (ESI): 378.3 [M+1].

### Step 7

### Methyl (E)-1-(2-chloro-4-fluorophenyl)-N-(6-methoxypyridin-3-yl)-3-azabicyclo[3.1.0]hexane-3-carbimidothioate 8i

The crude product **8h** (140 mg, 0.37 mmol) was added to 10 mL of tetrahydrofuran. The reaction solution was cooled in an ice bath, and added with potassium *tert*-butoxide (85 mg, 0.74 mmol). After completion of the addition, the reaction solution was stirred for 3 hours, and then added with methyl *p*-toluenesulfonate (155 mg, 0.81 mmol). After completion of the addition, the ice bath was removed. The reaction solution was naturally warmed up to room temperature, and stirred for 15 hours. The reaction solution was added with ice water (20 mL), and then extracted with ethyl acetate (30 mL×3). The organic phases were combined, washed with saturated sodium chloride solution (20 mL×2), dried over anhydrous sodium sulfate and filtrated to remove the drying agent. The filtrate was concentrated under reduced pressure, and the resulting residue was purified by thin layer chromatography with developing solvent system C to obtain the title product **8i** (50 mg), yield: 34.4%.
MS m/z (ESI): 392.3 [M+1].

### Step 8

### 1-(2-Chloro-4-fluorophenyl)-3-(5-(methoxymethyl)-4-(6-methoxypyridin-3-yl)-4H-1,2, 4-triazol-3-yl)-3-azabicyclo[3.1.0]hexane 8

**8i** (50 mg, 0.17 mmol), **4i** (88 mg, 0.85 mmol) and trifluoroacetic acid (two drops) were added to 5 mL of tetrahydrofuran. After completion of the addition, the reaction solution was heated to 70°C and stirred for 2 hours. After stopping heating, the reaction solution was concentrated under reduced pressure. The resulting residue was purified by thin layer chromatography with developing solvent system A to obtain the title product **8** (8 mg), yield: 10.9%.
MS m/z (ESI): 430.2 [M+1].
¹H NMR (400 MHz, CDCl₃): *δ* 8.17 (s, 1H), 7.56 (d, 1H), 7.27 (d, 1H), 7.10 (d, 1H), 6.91-6.88 (m, 2H), 4.28 (s, 2H), 4.01 (s, 3H), 3.64 (d, 1H), 3.46 (d, 2H), 3.43 (d, 1H), 3.28 (s, 3H), 1.73-1.70 (m, 1H), 1.01-0.98 (m, 2H).

### Example 9

### (1S,5R)-1-(2-Chloro-4-fluorophenyl)-3-(5-(methoxymethyl)-4-(6-methoxypyridin-3-yl)-4H-1,2,4-triazol-3-yl)-3-azabicyclo[3.1.0]hexane 9

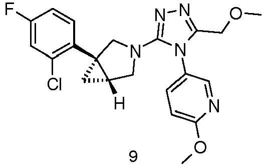

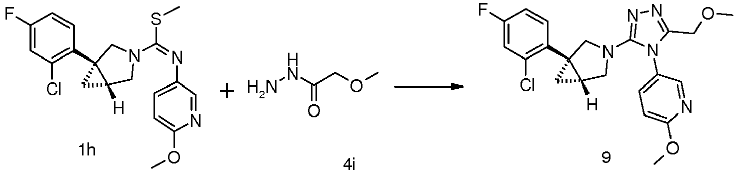

**1h** (180 mg, 0.46 mmol), **4i** (239 mg, 2.3 mmol) and trifluoroacetic acid (52 mg, 0.46 mmol) were added to 8 mL of tetrahydrofuran. After completion of the addition, the reaction solution was heated to 70°C and stirred for 3 hours. After stopping heating, the reaction solution was concentrated under reduced pressure. The resulting residue was purified by high performance liquid chromatography to obtain the title product **9** (50 mg), yield: 24.21%.
MS m/z (ESI):430.2 [M+1].
¹H NMR (400 MHz, CDCl₃): *δ* 8.22 (s, 1H), 7.66 (d, 1H), 7.27 (t, 1H), 7.10 (d, 1H), 6.91-6.89 (m, 2H), 4.24 (s, 2H), 4.01 (s, 3H), 3.78 (d, 1H), 3.57-3.55 (m, 2H), 3.53 (d, 1H), 3.25 (s, 3H), 1.81-1.79 (m, 1H), 1.10 (t, 1H), 0.95 (t, 1H).

### Example 10

### (1R,5S)-1-(2-Chloro-4-fluorophenyl)-3-(5-(methoxymethyl)-4-(6-methoxypyridin-3-yl)-4H-1,2,4-triazol-3-yl)-3-azabicyclo[3.1.0]hexane 10

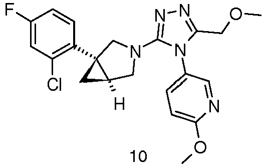

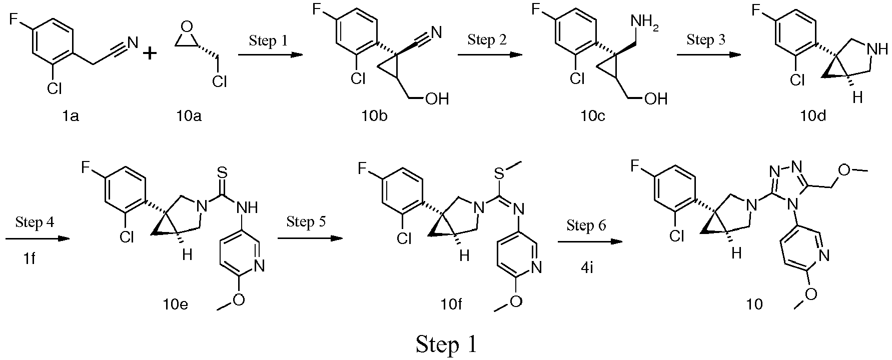

### (1R)-1-(2-Chloro-4-fluorophenyl)-2-(hydroxymethyl)cyclopropanecarbonitrile 10b

**1a** (1 g, 5.9 mmol) was dissolved in 8 mL of tetrahydrofuran. The reaction solution was cooled to -20°C in a dry ice-acetone bath, and added slowly with sodium bis(trimethylsilyl)amide (2.2 g, 11.8 mmol). After completion of the addition, the reaction solution was stirred for 30 minutes, and then added with (*S*)-2-(chloromethyl)oxirane **10a** (600 mg, 6.49 mmol). After completion of the addition, the dry ice-acetone bath was removed. The reaction solution was naturally warmed up to room temperature, and stirred for 3 hours. The reaction was quenched with saturated ammonium chloride solution (20 mL), and the reaction solution was extracted with ethyl acetate (50 mL×3). The organic phases were combined, and concentrated under reduced pressure to obtain the crude title product **10b** (1.3 g), which was used directly in the next step without purification.
MS m/z (ESI): 226.3 [M+1].

### Step 2

### ((2R)-2-(Aminomethyl)-2-(2-chloro-4-fluorophenyl)cyclopropyl)methanol 10c

Lithium aluminum hydride (210 mg, 5.5 mmol) was added to 8 mL of tetrahydrofuran. The reaction solution was cooled in an ice bath, and added with the crude product **10b** (500 mg, 2.22 mmol). After completion of the addition, the ice bath was removed. The reaction solution was naturally warmed up to room temperature, and stirred for 15 hours. The reaction was quenched by adding water (0.25 mL), sodium hydroxide solution (2 N, 0.25 mL) and water (0.75 mL) successively to the reaction solution. The reaction solution was filtrated, and the filtrate was concentrated under reduced pressure to obtain the crude title product **10c** (300 mg), which was used directly in the next step without purification.
MS m/z (ESI): 230.3 [M+1].

### Step 3

### (1R,5S)-1-(2-Chloro-4-fluorophenyl)-3-azabicyclo[3.1.0]hexane 10d

The crude product **10c** (505 mg, 2.2 mmol) was added to 8 mL of dichloromethane. The reaction solution was cooled in an ice bath, and added with thionyl chloride (393 mg, 3.3 mmol). After completion of the addition, the ice bath was removed, and the reaction solution was stirred for 3 hours. The reaction solution was concentrated under reduced pressure to obtain the crude title product **10d** (300 mg), which was used directly in the next step without purification.
MS m/z (ESI): 212.2 [M+1].

### Step 4

### (1R,5S)-1-(2-Chloro-4-fluorophenyl)-N-(6-methoxypyridin-3-yl)-3-azabicyclo[3.1.0]he xane-3-carbothioamide 10e

The 1**f** (366 mg, 2.2 mmol) and the crude product **10d** (233 mg, 1.1 mmol) were added to 8 mL of tetrahydrofuran. After completion of the addition, the reaction solution was stirred for 3 hours. The reaction solution was concentrated under reduced pressure to obtain the crude title product **10e** (260 mg), which was used directly in the next step without purification.
MS m/z (ESI): 378.2 [M+1].

### Step 5

### Methyl (1R,5S,E)-1-(2-chloro-4-fluorophenyl)-N-(6-methoxypyridin-3-yl)-3-azabicyclo[3.1.0]hexane-3-carbimidothioate 10f

The crude product **10e** (416 mg, 1.1 mmol) was added to 8 mL of tetrahydrofuran. The reaction solution was cooled in an ice bath, and added with potassium *tert*-butoxide (449 mg, 4 mmol). After completion of the addition, the reaction solution was stirred for 1 hour, and then added with methyl *p*-toluenesulfonate (410 mg, 2.2 mmol). After completion of the addition, the ice bath was removed. The reaction solution was naturally warmed up to room temperature, and stirred for 48 hours. The reaction solution was added with ice water (20 mL), and then extracted with ethyl acetate (50 mL×3). The organic phases were combined, washed with saturated sodium chloride solution (50 mL×3), and concentrated under reduced pressure. The resulting residue was purified by thin layer chromatography with developing solvent system A to obtain the title product **10f** (300 mg), yield: 62.6%.
MS m/z (ESI):392.3 [M+1].

### Step 6

### (1R,5S)-1-(2-Chloro-4-fluorophenyl)-3-(5-(methoxymethyl)-4-(6-methoxypyridin-3-yl)-4H-1,2,4-triazol-3-yl)-3-azabicyclo[3.1.0]hexane 10

**10f** (100 mg, 0.26 mmol), **4i** (133 mg, 1.28 mmol) and trifluoroacetic acid (29 mg, 0.26 mmol) were added to 5 mL of tetrahydrofuran. After completion of the addition, the reaction solution was heated to 70°C and stirred for 3 hours. After stopping heating, the reaction solution was concentrated under reduced pressure. The resulting residue was purified by thin layer chromatography with developing solvent system A, and then purified by high performance liquid chromatography to obtain the title product **10** (10 mg), yield: 9.0%.
MS m/z (ESI): 430.2 [M+1].
¹H NMR (400 MHz, CDCl₃): *δ* 8.16 (s, 1H), 7.56 (d, 1H), 7.27 (d, 1H), 7.09 (d, 1H), 6.87-6.85 (m, 2H), 4.27 (s, 2H), 4.00 (s, 3H), 3.66 (d, 1H), 3.45 (d, 2H), 3.36 (d, 1H), 3.27 (s, 3H), 1.72-1.70 (m, 1H), 0.98-0.96 (m, 2H).

### Example 11

### (1S,5R)-1-(2-Chloro-4-fluorophenyl)-3-(5-((difluoromethoxy)methyl)-4-(6-methoxypyri din-3-yl)-4H-1,2,4-triazol-3-l-3-azabicclo[3.1.0]hexane 11

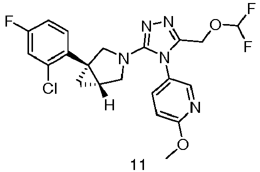

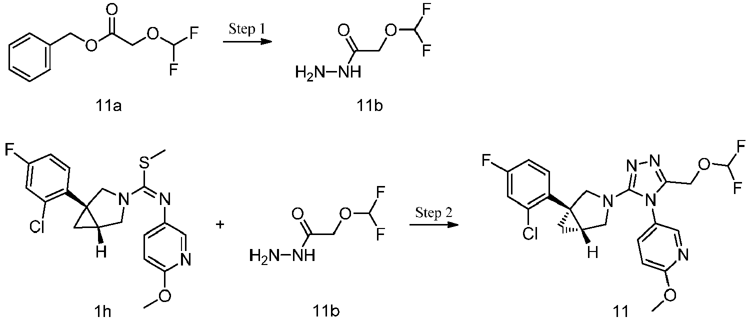

### Step 1

### 2-(Difluoromethoxy)acetohydrazide 11b

In accordance with the synthetic route of Example 1, compound **1k** in Step 7 was replaced with benzyl 2-(difluoromethoxy)acetate **11a** (prepared according to the method disclosed in the patent application "WO2015180612"), accordingly, the title compound **11b** (35 mg) was prepared.

### Step 2

### (1S,5R)-1-(2-Chloro-4-fluorophenyl)-3-(5-((difluoromethoxy)methyl)-4-(6-methoxypyri din-3-yl)-4H-1,2,4-triazol-3-yl)-3-azabicyclo[3.1.0]hexane 11

In accordance with the synthetic route of Example 1, compound **1l** in Step 8 was replaced with compound **11b,** accordingly, the title compound **11** (20 mg) was prepared, yield: 6.6%.
MS m/z (ESI): 466.4 [M+1].
¹H NMR (400 MHz, CD₃OD) *δ* 8.29 (d, 1H), 7.79 (dd, 1H), 7.43 (dd, 1H), 7.22 (dd, 1H), 7.04 (td, 1H), 7.00 (d, 1H), 6.35 (t, 1H), 4.78 (s, 2H), 4.01 (s, 3H), 3.66 (dd, 1H), 3.50 (d, 1H), 3.40 (d, 1H), 3.35 (d, 1H), 1.88-1.82 (m, 1H), 1.06-0.98 (m, 2H).

### Example 12

### (1S,5R)-1-(2-Chloro-4-fluorophenyl)-3-(4-(6-methoxypyridin-3-yl)-5-(trifluoromethyl)-4H-1,2,4-triazol-3-yl)-3-azabicyclo[3.1.0]hexane 12

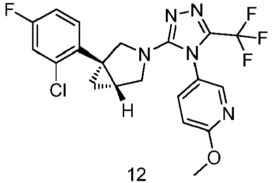

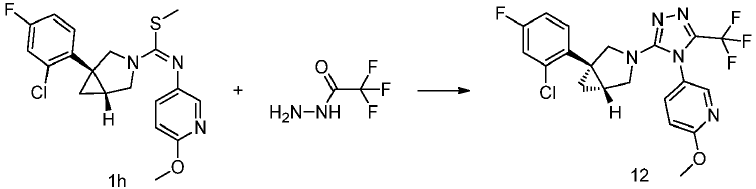

In accordance with the synthetic route of Example 1, compound **1l** in Step 8 was replaced with 2,2,2-trifluoroacetohydrazide (purchased from Shanghai Bide Pharmatech Ltd.), accordingly, the title compound **12** (30 mg) was prepared, yield: 51%.
MS m/z (ESI): 454.4 [M+1].
¹H NMR (400 MHz, CD₃OD) *δ* 8.35 (s, 1H), 7.87 (d, 1H), 7.44 (dd, 1H), 7.23 (dd, 1H), 7.04 (td, 1H), 7.00 (d, 1H), 4.02 (s, 3H), 3.70 (dd, 1H), 3.54 (d, 1H), 3.43 (d, 1H), 3.38 (d, 1H), 1.89-1.84 (m, 1H), 1.07 (dd, 1H), 0.98 (t, 1H).

### Example 13

### (1S,5R)-1-(2-Chloro-4-fluorophenyl)-3-(5-(difluoromethyl)-4-(6-methoxypyridin-3-yl)-4H-1,2,4-triazol-3-yl)-3-azabicyclo[3.1.0]hexane 13

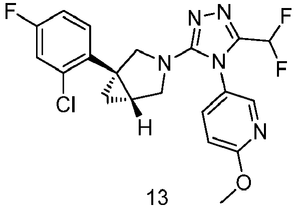

In accordance with the synthetic route of Example 1, compound **1l** in Step 8 was replaced with 2,2-difluoroacetohydrazide (prepared according to the method disclosed in the patent application "US6979686"), accordingly, the title compound **13** (12 mg) was prepared, yield: 10.8%.
MS m/z (ESI): 436.1 [M+1].
¹H NMR (400 MHz, CDCl₃) *δ* 8.21 (s, 1H), 7.81 (d, 1H), 7.35 (m, 1H), 7.18-7.04 (m, 1H), 6.99-6.79 (m, 2H), 6.78-6.52 (m, 1H), 4.02 (s, 3H), 3.70 (dd, 1H), 3.49-3.42 (m, 2H), 3.41 (d, 1H), 1.89-1.82 (m, 1H), 1.07 (dd, 1H), 0.98 (t, 1H).

### Example 14

### (1S,5R)-1-(2-Chloro-4-fluorophenyl)-3-(5-((methoxy-d₃)methyl)-4-(6-methoxypyridin-3-yl)-4H-1,2,4-triazol-3-yl)-3-azabicyclo[3.1.0]hexane 14

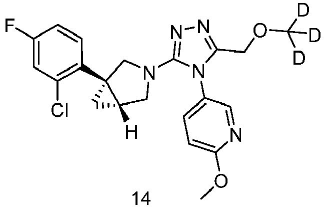

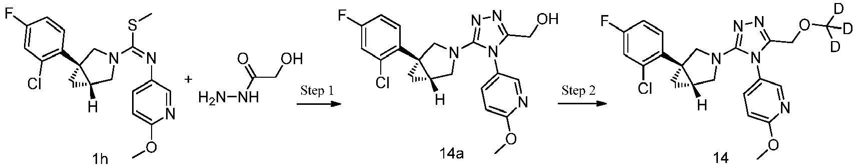

### Step 1

### (1S,5R)-1-(2-Chloro-4-fluorophenyl)-3-(5-(hydroxymethyl)-4-(6-methoxypyridin-3-yl)-4H-1,2,4-triazol-3-yl)-3-azabicyclo[3.1.0]hexane 14a

In accordance with the synthetic route of Example 1, compound **1l** in Step 8 was replaced with 2-hydroxyacetohydrazide (prepared according to the method disclosed in the patent application "WO2008051493"), accordingly, the title compound **14a** (90 mg) was prepared, yield: 68%.
MS m/z (ESI): 416.4 [M+1].
¹H NMR (400 MHz, CD₃OD) *δ* 8.29 (d, 1H), 7.80 (dd, 1H), 7.42 (dd, 1H), 7.22 (dd, 1H), 7.03 (td, 1H), 6.99 (d, 1H), 4.42 (s, 2H), 4.02 (s, 3H), 3.64 (dd, 1H), 3.47 (d, 1H), 3.38 (d, 1H), 3.34 (d, 1H), 1.87-1.80 (m, 1H), 1.01 (d, 2H).

### Step 2

### (1S,5R)-1-(2-Chloro-4-fluorophenyl)-3-(5-((methoxy-d₃)methyl)-4-(6-methoxypyridin-3-yl)-4H-1,2,4-triazol-3-yl)-3-azabicyclo[3.1.0]hexane 14

**14a** (90 mg, 0.22 mmol) and *N*,*N*-dimethylformamide (5 mL) were added to a reaction flask under an argon atmosphere. The reaction solution was added with sodium hydride (16 mg, 0.65 mmol) in an ice bath, and stirred for 10 minutes. The reaction solution was added with deuterated iodomethane (156 mg, 1.08 mmol), and warmed up to room temperature for 3 hours. The reaction solution was added with water (15 mL), and then extracted with ethyl acetate (10 mL×3). The organic phases were combined, dried over anhydrous sodium sulfate and concentrated under reduced pressure. The resulting residue was purified by silica gel column chromatography with elution system C to obtain the title product **14** (10 mg), yield: 10.8%.
MS m/z (ESI): 433.2 [M+1].
¹H NMR (400 MHz, CDCl₃) *δ* 8.17 (d, 1H), 7.56 (d, 1H), 7.28 (t, 1H), 7.09 (d, 1H), 6.87 (d, 1H), 6.85 (d, 1H), 4.27 (s, 2H), 4.00 (s, 3H), 3.67 (d, 1H), 3.46 (d, 2H), 3.36 (d, 1H), 1.73-1.70 (m, 1H), 0.98-0.95 (m, 2H).

### Example 15

### (1S,5R)-1-(2-Chloro-4-fluorophenyl)-3-(4-(6-methoxypyridin-3-yl)-5-((((R)-tetrahydrof uran-3-yl)oxy)methyl)-4H-1,2,4-triazol-3-yl)-3-azabicyclo[3.1.0]hexane 15

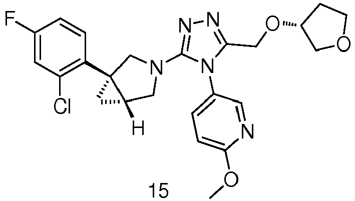

In accordance with the synthetic route of Example 3, compound **3a** in Step 1 was replaced with (*R*)-3-hydroxytetrahydrofuran (purchased from Shanghai Bide Pharmatech Ltd.), accordingly, the title compound **15** (30 mg) was prepared, yield: 13.2%.
MS m/z (ESI): 486.4 [M+1].
¹H NMR (400 MHz, CD₃OD) *δ* 8.20 (d, 1H), 7.59 (dd, 1H), 7.32-7.28 (m, 1H), 7.14 (dd, 1H), 7.11 (td, 1H), 6.98 (d, 1H), 4.40-4.33 (m, 2H) 4.17-4.15 (m, 1H), 4.03 (s, 3H), 3.81-3.79 (m, 2H), 3.68-3.65 (m, 3H), 3.50 (d, 2H), 3.40 (d, 1H), 1.98-1.90 (m, 1H), 1.89-1.81 (m, 1H), 1.79-1.74 (m, 1H), 1.02-0.92 (m, 2H).

### Example 16

### (1S,5R)-1-(2-Chloro-4-fluorophenyl)-3-(4-(6-methoxypyridin-3-yl)-5-cyanomethyl-4H-1,2,4-triazol-3-yl)-3-azabicyclo[3.1.0]hexane 16

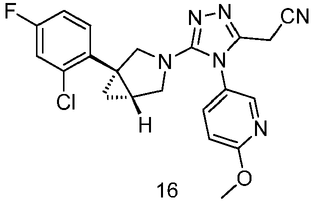

In accordance with the synthetic route of Example 1, compound **1l** in Step 8 was replaced with 2-cyanoacetohydrazide (purchased from Shanghai Bide Pharmatech Ltd.), accordingly, the title compound **16** (30 mg) was prepared, yield: 24.3%.
MS m/z (ESI): 425.4 [M+1].
¹H NMR (400 MHz, CD₃OD) *δ* 8.21 (d, 1H), 7.63 (dd, 1H), 7.33-7.30 (m, 1H), 7.15 (dd, 1H), 7.00 (d, 1H), 6.98 (td, 1H), 4.06 (s, 3H), 3.72-3.69 (m, 3H), 3.49 (d, 2H), 3.42 (d, 1H), 1.80-1.76 (m, 1H), 1.06-0.98 (m, 2H).

### Example 17

### (1S,5R)-1-(2-Chloro-4-fluorophenyl)-3-(5-cyclopropyl-4-(6-methoxypyridin-3-yl)-4H-1 ,2,4-triazol-3-yl)-3-azabicyclo[3.1.0]hexane 17

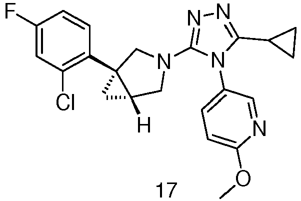

In accordance with the synthetic route of Example 1, compound **1l** in Step 8 was replaced with 2-cyclopropylformohydrazide (purchased from Shanghai Bide Pharmatech Ltd.), accordingly, the title compound **17** (30 mg) was prepared, yield: 27.5%.
MS m/z (ESI): 426.2 [M+1].
¹H NMR (400 MHz, CD₃OD) *δ* 8.24 (d, 1H), 7.67 (dd, 1H), 7.30-7.25 (m, 1H), 7.17 (dd, 1H), 6.94 (d, 1H), 6.90 (td, 1H), 4.06 (s, 3H), 3.75 (dd, 1H), 3.55-3.47 (m, 2H), 3.45 (d, 1H), 1.79-1.72 (m, 1H), 1.43-1.36 (m, 1H), 1.15-1.10 (m, 2H), 1.06-0.96 (m, 2H), 0.89-0.86 (m, 2H).

### Test Examples:

### Biological Assay

### Test Example 1. Determination of the inhibition activity of the compounds of the present invention on human OTR

The inhibition effect of the compounds of the present invention on the activity of human OTR protein expressed in HEK293/human OTR stably transfected cells was determined by the following experimental method:
I. Experimental materials and instruments
   1. Fluo-4 NW calcium assay kit (F36206, invitrogen)
   2. MEM (Hyclone, SH30024.01B)
   3. G418 sulfate (Enzo, ALX-380-013-G005)
   4. Fetal bovine serum (GIBCO, 10099)
   5. Sodium pyruvate solution (sigma, S8636-100ML)
   6. MEM non-essential amino acid solution (100×) (sigma, M7145-100ML)
   7. Flexstation 3 multi-function microplate reader (Molecular Devices)
   8. Poly-D-lysine 96-well plate, black/clear (356692, BD)
   9. Oxytocin (synthesized by GL Biochem Ltd.)
   10. pcDNA3.1 (invitrogen, V79020)
   11. pcDNA3.1-hOTR (NM-000916) (synthesized and constructed into pcDNA3.1 plasmid by GENEWIZ Biological Technology Co., Ltd)
   12. HEK293 cells (Cat. No. GNHu18, Cell bank of Chinese Academy of Sciences)
II. Experimental procedures

The pcDNA3.1-hOTR plasmid was transferred into HEK293 cells with the Lipofectamine® 3000 transfection reagent; G418 was added on the next day to screen, and monoclonal cell lines were selected.

HEK293/human OTR stably transfected cells were inoculated in a 96-well plate with an inoculation density of 25,000 cells/well one day in advance. On the next day, a loading buffer containing Fluo-4 dye was formulated using the reagents in the Fluo-4 NW calcium assay kit, and the culture medium was then removed; 100 µl of the loading buffer containing Fluo-4 dye were added to each well, and the plate was incubated at 37°C for 30 minutes. After that, the plate was moved to room temperature and equilibrated for 10 minutes. The compounds were formulated into concentration gradients of 10⁶, 10⁵, 10⁴, 10³, 10² and 10¹ nM. 1 µl of the compounds in each concentration was added to each well, and the plate was incubated at room temperature for 10 minutes. 50 µl of oxytocin polypeptide (3 nM) were automatically added by the machine, and the values were immediately detected at 494/516 nM by the flexstation 3 microplate reader. IC₅₀ values of the compounds were calculated by Graphpad prism software using fluorescence signals corresponding to different concentrations.

The inhibition activity of the compounds of the present invention on human OTR was determined by the above test, and the obtained IC₅₀ values are shown in Table 1.

**Table 1 IC₅₀ of inhibition activity of the compounds of the present invention on human OTR**

| Example No. | IC₅₀ (nM) |
|---|---|
| 1 | 44 |
| 4 | 29 |
| 5 | 131 |
| 6 | 4.3 |
| 8 | 23 |
| 9 | 14 |
| 10 (control) | 624 |
| 11 | 1 |
| 12 | 30 |
| 13 | 52 |
| 14 | 68 |

Conclusion: The compounds of the present invention have a significant inhibition effect on the human OTR activity.

### Test Example 2. Determination of the inhibition activity of the compounds of the present invention on human V1aR

The inhibition effect of the compounds of the present invention on the activity of human VlaR protein expressed in HEK293/human VlaR stably transfected cells was determined by the following experimental method:
I. Experimental materials and instruments
   1. Fluo-4 NW calcium assay kit (F36206, invitrogen)
   2. MEM (Hyclone, SH30024.01B)
   3. G418 sulfate (Enzo, ALX-380-013-G005)
   4. Fetal bovine serum (GIBCO, 10099)
   5. Sodium pyruvate solution (sigma, S8636-100ML)
   6. MEM non-essential amino acid solution (100×) (sigma, M7145-100ML)
   7. Flexstation 3 multi-function microplate reader (Molecular Devices)
   8. Poly-D-lysine 96-well plate, black/clear (356692, BD)
   9. Vasopressin (Tocris, 2935)
   10. pcDNA3.1 (invitrogen, V79020)
   11. pcDNA3.1-V1aR (NM-000706) (synthesized and constructed into pcDNA3.1 plasmid by GENEWIZ Biological Technology Co., Ltd)
   12. HEK293 cells (Cat. No. GNHu18, Cell Bank of Chinese Academy of Sciences)
II. Experimental procedures

The pcDNA3.1-V1aR plasmid was transferred into HEK293 cells with the Lipofectamine® 3000 transfection reagent; G418 was added on the next day to screen, and monoclonal cell lines were selected.

HEK293/human VlaR stably transfected cells were inoculated in a 96-well plate with an inoculation density of 25,000 cells/well one day in advance. On the next day, a loading buffer containing Fluo-4 dye was formulated using the reagents in the Fluo-4 NW calcium assay kit, and the culture medium was then removed; 100 µl of the loading buffer containing Fluo-4 dye were added to each well, and the plate was incubated at 37°C for 30 minutes. After that, the plate was moved to room temperature and equilibrated for 10 minutes. The compounds were formulated into concentration gradients of 10⁶, 10⁵, 10⁴, 10³, 10² and 10¹ nM. 1 µl of the compounds in each concentrationt was added to each well, and the plate was incubated at room temperature for 10 minutes. 50 µl of vasopressin polypeptide (3 nM) were automatically added by the machine, and the values were immediately detected at 494/516 nM by the flexstation 3 microplate reader. IC₅₀ values of the compounds were calculated by Graphpad prism software using fluorescence signals corresponding to different concentrations.

The inhibition activity of the compounds of the present invention on human VlaR was determined by the above test, and the obtained IC₅₀ values are shown in Table 2.

**Table 2 IC₅₀ of inhibition activity of the compounds of the present invention on human VlaR**

| Example No. | IC₅₀ (µM) |
|---|---|
| 1 | 1 |
| 2 | 4.7 |
| 3 | 7.5 |
| 4 | 3.8 |
| 5 | 1 |
| 6 | 1.7 |
| 7 | 2.0 |
| 8 | 3.0 |
| 9 | 2.9 |
| 12 | 3.6 |
| 13 | 1.9 |

Conclusion: The compounds of the present invention have a weak inhibition effect on the human VlaR activity, indicating that the compounds of the present invention have a selective inhibition effect on the OTR activity.

### Test Example 3. Determination of the inhibition activity of the compounds of the present invention on human V1bR

The inhibition effect of the compounds of the present invention on the activity of human V1bR protein expressed in HEK293/human V1bR cells was determined by the following experimental method:
I. Experimental materials and instruments
   1. Fluo-4 NW calcium assay kit (F36206, invitrogen)
   2. MEM (Hyclone, SH30024.01B)
   3. G418 sulfate (Enzo, ALX-380-013-G005)
   4. Fetal bovine serum (GIBCO, 10099)
   5. Sodium pyruvate solution (sigma, S8636-100ML)
   6. MEM non-essential amino acid solution (100×) (sigma, M7145-100ML)
   7. Flexstation 3 multi-function microplate reader (Molecular Devices)
   8. Poly-D-lysine 96-well plate, black/clear (356692, BD)
   9. Vasopressin (Tocris, 2935)
   10. pcDNA3.1 (invitrogen, V79020)
   11. pcDNA3.1-V1bR (NM-000706) (synthesized and constructed into pcDNA3.1 plasmid by GENEWIZ Biological Technology Co., Ltd)
   12. HEK293 cells (Cat. No. GNHu18, Cell Bank of Chinese Academy of Sciences)
II. Experimental procedures

The pcDNA3.1-V1bR plasmid was transferred into HEK293 cells with the Lipofectamine® 3000 transfection reagent; G418 was added on the next day, and the HEK293/human V1bR pool cell lines were obtained.

HEK293/human V1bR pool cells were inoculated in a 96-well plate with an inoculation density of 25,000 cells/well one day in advance. On the next day, a loading buffer containing Fluo-4 dye was formulated using the reagents in the Fluo-4 NW calcium assay kit, and the culture medium was then removed; 100 µl of the loading buffer containing Fluo-4 dye were added to each well, and the plate was incubated at 37°C for 30 minutes. After that, the plate was moved to room temperature and equilibrated for 10 minutes. The compounds were formulated into concentration gradients of 10⁶, 10⁵, 10⁴, 10³, 10² and 10¹ nM. 1 µl of the compounds in each concentration was added to each well, and the plate was incubated at room temperature for 10 minutes. 50 µl of vasopressin polypeptide (3 nM) were automatically added by the machine, and the values were immediately detected at 494/516 nM by the flexstation 3 microplate reader. IC₅₀ values of the compounds were calculated by Graphpad prism software using fluorescence signals corresponding to different concentrations.

The inhibition activity of the compounds of the present invention on human V1bR was determined by the above test, and the obtained IC₅₀ values are shown in Table 3.

**Table 3 IC₅₀ of inhibition of the compounds of the present invention on human V1bR activity**

| Example No. | IC₅₀ (µM) |
|---|---|
| 1 | 24.5 |
| 3 | 56.7 |
| 4 | 59.4 |
| 6 | 27.9 |
| 7 | 12.4 |
| 8 | 43.3 |
| 9 | 37.6 |
| 11 | 7.8 |
| 12 | 11.7 |
| 14 | 20.5 |

Conclusion: The compounds of the present invention have no significant inhibition effect on the human V1bR activity, indicating that the compounds of the present invention have a selective inhibition effect on the OTR activity.

### Test Example 4. Determination of the inhibition activity of the compounds of the present invention on human V2R

The inhibition effect of the compounds of the present invention on the activity of human V2R protein expressed in HEK293/human V2R cells was determined by the following experimental method:
I. Experimental materials and instruments
   1. cAMP dynamic 2 kit - 1,000 tests (62AM4PEB, Cisbio)
   2. MEM (Hyclone, SH30024.01B)
   3. G418 sulfate (Enzo, ALX-380-013-G005)
   4. Fetal bovine serum (GIBCO, 10099)
   5. Sodium pyruvate solution (sigma, S8636-100ML)
   6. MEM non-essential amino acid solution (100×) (sigma, M7145-100ML)
   7. PheraStar multi-function microplate reader (BMG)
   8. Corning/Costar 384-well non-adsorbing microplate - black NBS plate (4514, Corning)
   9. Cell dissociation solution, enzyme-free, PBS (13151014-100ml, Thermo Fisher Scientific)
   10. HBSS, calcium, magnesium, no phenol red (14025-092, Invitrogen)
   11. HEPES, 1M buffer (15630-080, GIBCO)
   12. BSA (0219989725, MP Biomedicals)
   13. IBMX (I7018-250MG, sigma)
   14. Vasopressin (Tocris, 2935)
   15. pcDNA3.1 (invitrogen, V79020)
   16. pcDNA3.1-V2R (NM-000054) (synthesized and constructed into pcDNA3.1 plasmid by GENEWIZ Biological Technology Co., Ltd)
   17. HEK293 cells (Cat. No. GNHu18, Cell Bank of Chinese Academy of Sciences)
II. Experimental procedures

The pcDNA3.1-V2R plasmid was transferred into HEK293 cells with the Lipofectamine® 3000 transfection reagent; G418 was added on the next day, and the HEK293/human V2R pool cell lines were obtained.

### 1) Digestion of the cells:

HEK293/human V2R pool cells were digested with the cell dissociation solution (enzyme-free), thereby dissociating the cells from the cell culture dish into individual cells. After completion, the cell solution was blown well, and centrifuged to remove the supernatant. The cells were re-suspended in the test buffer 1 (1x HBSS+20mM HEPES+0.1%BSA) and counted. The cell density was adjusted to 1250 cells/5 µl, i.e., 2.5*10⁵/ml.

### 2) Formulation of the compounds

The compounds were formulated into a series of concentrations of 20 mM, 6.67 mM, 2.22 mM, 0.74 mM, 0.25 mM, 0.08 mM, 27.4 µM, 9.14 µM, 3.05 µM, 1.02 µM, 0.34 µM and 0 µM (DMSO) with pure DMSO. The compounds were then formulated into a 4-fold use concentration with the test buffer 2 (test buffer 1 + 1 mM IBMX).

Agonist: 460 µM vasopressin was used as the mother liquor, formulated as a 2 µM solution with DMSO, which was then diluted as a 0.5 nM solution with the test buffer 2.

Standard: The first point was 20 µl of a stock solution (2848 nM), which was diluted successively to a total of eleven concentrations in 4 fold concentration gradient with the test buffer 1 from the second point.

### 3) Addition of the compounds and incubation:

1. The well-mixed cells were added to a 384-well plate (5 µl/well) without changing the tip.
2. The test compounds and positive compound formulated were added (2.5 µl/well), and the tips should be changed.
3. The plate was centrifuged at 1000 rpm for 1 min, shaken for 30 sec to mix well, and incubated at room temperature for 30 min.
4. The standard curve wells were added with the test buffer 2 (5 µl/well).
5. The agonist formulated was added (2.5 µl/well), and the tips should be changed; the plate was centrifuged at 1000 rpm for 1 min, shaken for 30 sec to mix well, and incubated at room temperature for 30 min.
6. cAMP-d2 (a component in the cAMP dynamic 2 kit) and Anti-cAMP-Eu-Cryptate (a component in the cAMP dynamic 2 kit) were formulated in the dark, which were then mixed well with cAMP lysate (a component in the cAMP dynamic 2 kit) in a ratio of 1:4. Each well was added with the formulated cAMP-d2 solution (5 µl/well), followed by addition of Anti-cAMP-Eu-Cryptate (5 µl/well). The plate was shaken for 30 sec to mix well, and incubated in the dark at room temperature for 1h.

4) Plate reading: The HTRF signals were read by the PheraStar multi-function microplate reader.

### 5) Data processing

The data in this test was processed using the data processing software Graphpad Prism.

The inhibition activity of the compounds of the present invention on human V2R was determined by the above test, and the obtained IC₅₀ values are shown in Table 4.

**Table 4 IC₅₀ of inhibition activity of the compounds of the present invention on human V2R**

| Example No. | IC₅₀ (µM) |
|---|---|
| 1 | 21.4 |
| 3 | 23.0 |
| 4 | 25.7 |
| 6 | 7.3 |
| 7 | 90 |
| 9 | 32.6 |
| 11 | 6.8 |
| 12 | 29.5 |
| 14 | 10.4 |

Conclusion: The compounds of the present invention have no significant inhibition effect on the human V2R activity, indicating that the compounds of the present invention have a selective inhibition effect on the OTR activity.

### Pharmacokinetics Evaluation

### Test Example 5. Pharmacokinetics assay of the compounds of the present invention

### 1. Abstract

Rats were used as test animals. The drug concentration in plasma at different time points was determined by LC/MS/MS method after intragastrical administration of the compounds of Examples 6, 8, 9 and 11 to rats. The pharmacokinetic behavior of the compounds of the present invention was studied and evaluated in rats.

### 2. Test protocol

### 2.1 Test compounds

Compounds of Examples 6, 8, 9 and 11.

### 2.2 Test animals

Sixteen healthy adult Sprague-Dawley (SD) rats (half male and half female) were purchased from Shanghai Jiesijie Laboratory Animal Co., LTD, with Certificate No.: SCXK (Shanghai) 2013-0006, and equally divided into 4 groups (4 rats per group).

### 2.3 Preparation of the test compounds

A certain amount of the test compound was weighed, and added with 2.5% by volume of DMSO and 97.5% by volume of 10% solutol HS-15 to prepare a 0.2 mg/mL colorless, clear and transparent solution.

### 2.4 Administration

After an overnight fast, SD rats were administered intragastrically the test compounds at an administration dosage of 2.0 mg/kg and an administration volume of 10.0 mL/kg.

### 3. Process

The rats were intragastrically administered the compounds of Examples 6, 8, 9 and 11. 0.2 mL of blood was taken from the orbital sinus before administration and at 0.5, 1.0, 2.0, 4.0, 6.0, 8.0, 11.0 and 24.0 hours after administration. The samples were stored in heparinized tubes, and centrifuged for 10 minutes at 4°C at 3,500 rpm to separate the blood plasma. The plasma samples were stored at -20°C. The rats were fed 2 hours after administration.

The content of the test compounds in the plasma of rats after intragastrical administration of the test compounds at different concentrations was determined: 25 µL of rat plasma at each time after administration was taken, added with 50 µL of the internal standard solution of camptothecin (100 ng/mL) and 200 µL of acetonitrile, vortex-mixed for 5 minutes, and centrifuged for 10 minutes (4000 rpm). 1.0 µL of the supernatant was taken from the plasma samples for LC/MS/MS analysis.

### 4. Results of pharmacokinetic parameters

Pharmacokinetic parameters of the compounds of the present invention are shown below:

| No. | Pharmacokinetics assay (2 mg/kg) | | | | | |
|---|---|---|---|---|---|---|
| | Plasma concentration | Area under curve | Half-life | Residence time | Clearance | Apparent distribution volume |
| | Cmax (ng /mL) | AUC (ng /mL*h) | T1/2 (h) | MRT (h) | CLz/F (ml/min/kg) | Vz/F (ml/kg) |
| Example 6 | 135±80.0 | 1057±982 | 3.47±1.72 | 6.70±0.73 | 68.9±58.8 | 18032±17374 |
| Example 8 | 635±273 | 1985±1021 | 1.08±0.21 | 2.18±0.24 | 23.3±18.0 | 1941±983 |
| Example 9 | 350±142 | 2042±1025 | 3.98±3.55 | 6.23±4.94 | 20.2±10.3 | 6004±4440 |
| Example 11 | 405±110 | 3985±2970 | 2.86±1.27 | 5.82±2.73 | 12.6±8.03 | 2465±706 |

Conclusion: The compounds of the present invention are well absorbed, and have a pharmacokinetic advantage.

## Claims

1. A compound of formula (I): 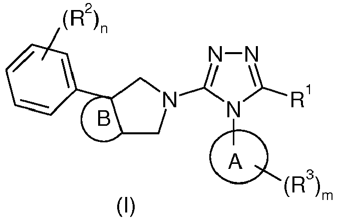 or a tautomer, mesomer, racemate, enantiomer, diastereomer thereof, or mixture thereof, or a pharmaceutically acceptable salt thereof,
wherein:
ring A is aryl or heteroaryl;
ring B is cycloalkyl or heterocyclyl;
R¹ is alkyl or cycloalkyl, wherein the alkyl is optionally substituted by one or more substituents selected from the group consisting of alkoxy, halogen, haloalkyl, haloalkoxy, deuterated alkoxy, hydroxy, hydroxyalkyl, cyano, amino, nitro, cycloalkyl, heterocyclyl, heterocyclyloxy, aryl, heteroaryl and -OR⁴;
each R² is identical or different and each is independently selected from the group consisting of hydrogen, halogen, alkyl, alkoxy, haloalkyl, hydroxy, hydroxyalkyl, cyano, amino, nitro, cycloalkyl and heterocyclyl;
each R³ is identical or different and each is independently selected from the group consisting of hydrogen, halogen, alkyl, alkoxy, haloalkyl, hydroxy, hydroxyalkyl, cyano, amino, nitro, cycloalkyl and heterocyclyl;
R⁴ is selected from the group consisting of hydroxyalkyl, cycloalkyl, aryl and heteroaryl;
n is 0, 1, 2, 3, 4 or 5; and
m is 0, 1, 2, 3 or 4.

2. The compound of formula (I) according to claim 1, being a compound of formula (II): 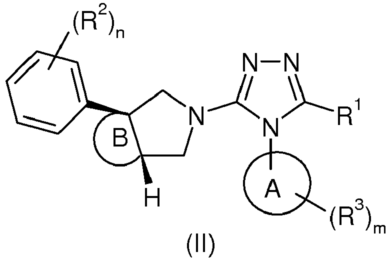 or a tautomer, mesomer, racemate, enantiomer, diastereomer thereof, or mixture thereof, or a pharmaceutically acceptable salt thereof,
ring A, ring B, R¹-R³, n and m are as defined in claim 1.

3. The compound of formula (I) according to claim 1 or 2, wherein ring B is 3-5 membered cycloalkyl or heterocyclyl, and preferably cyclopropyl.

4. The compound of formula (I) according to any one of claims 1 to 3, being a compound of formula (III): 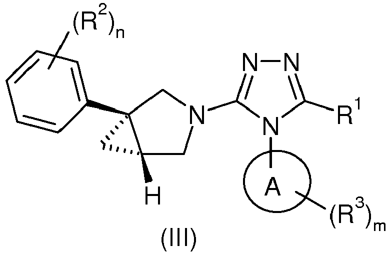 or a tautomer, mesomer, racemate, enantiomer, diastereomer thereof, or mixture thereof, or a pharmaceutically acceptable salt thereof,
wherein:
ring A, R¹-R³, n and m are as defined in claim 1.

5. The compound of formula (I) according to any one of claims 1 to 4, wherein ring A is pyridyl or benzodioxol, and preferably

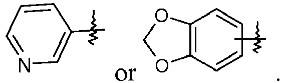

6. The compound of formula (I) according to any one of claims 1 to 5, wherein R¹ is alkyl or cycloalkyl, wherein the alkyl is optionally substituted by one or more substituents selected from the group consisting of halogen, cyano, alkoxy, haloalkoxy, deuterated alkoxy and heterocyclyloxy.

7. The compound of formula (I) according to any one of claims 1 to 6, wherein each R² is identical or different and each is independently selected from the group consisting of hydrogen, halogen and alkyl.

8. The compound of formula (I) according to any one of claims 1 to 7, wherein R³ is alkoxy.

9. The compound of formula (I) according to any one of claims 1 to 8, wherein n is 2; and m is 0 or 1.

10. The compound according to any one of claims 1 to 9, selected from the group consisting of:

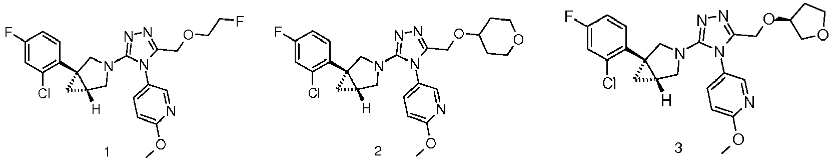

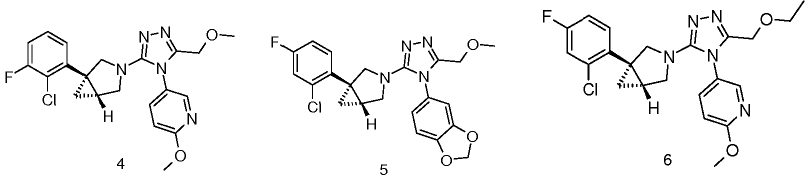

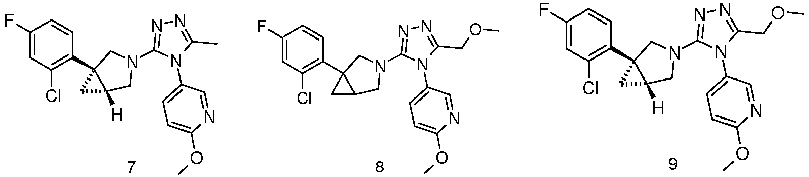

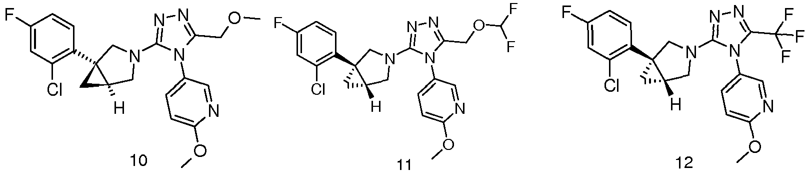

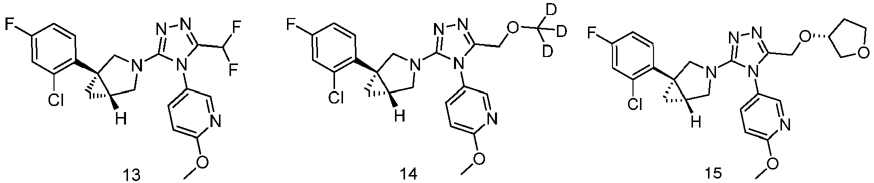

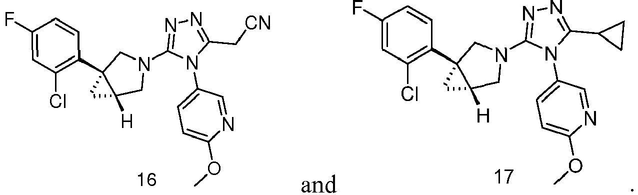

11. A compound of formula (I-A): 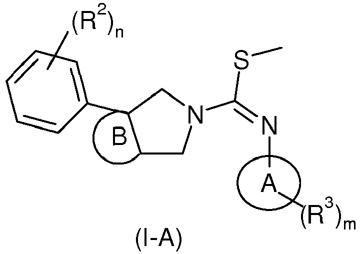 or a tautomer, mesomer, racemate, enantiomer, diastereomer thereof, or mixture thereof, or a pharmaceutically acceptable salt thereof,
wherein:
ring A, ring B, R², R³, n and m are as defined in claim 1.

12. The compound of formula (I-A) according to claim 9, selected from the group consisting of:

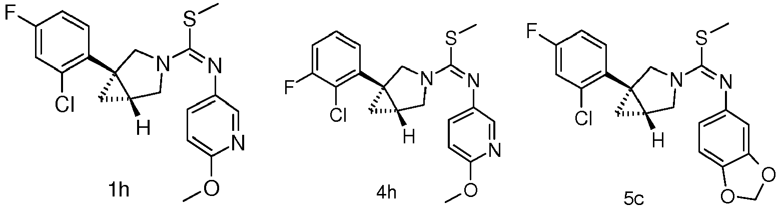

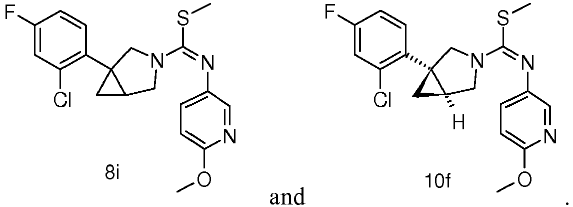

13. A method for preparing the compound of formula (I) according to claim 1, comprising a step of: 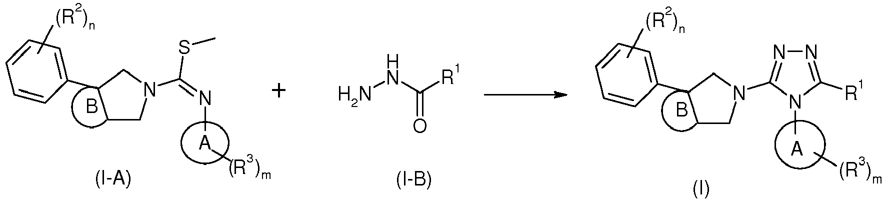 heating a compound of formula (I-A) and a compound of formula (I-B) or a hydrochloride salt thereof under an acidic condition to obtain the compound of formula (I),
wherein:
ring A, ring B, R¹-R³, n and m are as defined in claim 1.

14. A pharmaceutical composition, comprising a therapeutically effective amount of the compound of formula (I), or a tautomer, mesomer, racemate, enantiomer, diastereomer thereof, or mixture thereof, or a pharmaceutically acceptable salt thereof according to any one of claims 1 to 10, and one or more pharmaceutically acceptable carriers, diluents or excipients.

15. Use of the compound of formula (I), or a tautomer, mesomer, racemate, enantiomer, diastereomer thereof, or mixture thereof, or a pharmaceutically acceptable salt thereof according to any one of claims 1 to 10, or the pharmaceutical composition according to claim 14 in the preparation of a medicament for treating or preventing a disease or condition where inhibition of oxytocin is known, or can be shown, to produce a beneficial effect.

16. The use according to claim 15, wherein the disease or condition where inhibition of oxytocin is known, or can be shown, to produce a beneficial effect is selected from the group consisting of sexual dysfunction, male sexual dysfunction, female sexual dysfunction, hypoactive sexual desire disorder, sexual arousal disorder, orgasmic disorder, sexual pain disorder, premature ejaculation, preterm labour, complications in labour, appetite and feeding disorders, benign prostatic hyperplasia, premature birth, dysmenorrhea, congestive heart failure, arterial hypertension, liver cirrhosis, nephrotic hypertension, ocular hypectension, obsessive compulsive disorder and neuropsychiatric disorders, and preferably selected from the group consisting of sexual arousal disorder, orgasmic disorder, sexual pain disorder and premature ejaculation.

17. Use of the compound of formula (I), or a tautomer, mesomer, racemate, enantiomer, diastereomer thereof, or mixture thereof, or a pharmaceutically acceptable salt thereof according to any one of claims 1 to 10, or the pharmaceutical composition according to claim 14 in the preparation of a medicament for antagonizing oxytocin.
